# EUROPEAN PATENT SPECIFICATION

(11) **EP 1 948 785 B1**
(45) Date of publication and mention of the grant of the patent: **15.01.2014**
(21) Application number: 06839527.6
(22) Date of filing: 24.10.2006
(51) Int. Cl.: C12N 5/07, C12P 21/06, C12N 5/16

(54) **GLP-2 MIMETIBODIES, POLYPEPTIDES, COMPOSITIONS, METHODS AND USES**
GLP-2-MIMETIKÖRPER, POLYPEPTIDE, ZUSAMMENSETZUNGEN, VERFAHREN UND VERWENDUNGEN
CORPS MIMETIQUES ET POLYPEPTIDES DE GLP-2, COMPOSITIONS, METHODES ET UTILISATIONS

(30) Priority: 24.10.2005 US 729704 P; 31.08.2006 US 824160 P; 23.10.2006 US 862487 P
(43) Date of publication of application: 30.07.2008
(73) Proprietor: Janssen Biotech, Inc., Horsham, PA 19044 (US)
(72) Inventor: BAKER, Audrey, E., Philadelphia, PA 19119 (US); MOORE, Beverly, A., North Wales, PA 19454 (US); NESSPOR, Thomas, Collegeville, PA 19426 (US); O'NEIL, Karyn, Media, PA 19063 (US); PALMER, Jeffrey, M., Chalfont, PA 18914 (US); PICHA, Kristen, Malvern, PA 19355 (US); SAGUE, Sarah, Malvern, PA 19355 (US)
(74) Representative: Marshall, Cameron John
(86) International application number: PCT/US2006/060197
(87) International publication number: WO 2007/067828

(56) References cited:
- WO-A-2005/097175
- WO-A2-2005/097175
- US-A1- 2001 021 767
- US-A1- 2001 021 767
- US-A1- 2004 122 210
- US-A1- 2004 198 642
- US-A1- 2005 136 061
- US-A1- 2005 191 301

## Description

### Field of the Invention

The present invention relates to mammalian GLP-2 polypeptides and mimetibodies, and their use as therapeutics.

### Background of the Invention

Glucagon-like peptide-2 (GLP-2) is a 33 amino acid intestinotrophic peptide hormone generated via post-translational processing of proglucagon (Orskov et al., FEBS Lett. 247: 193-196 (1989); Hartmann et al., Peptides 21: 73-80 (2000)). In mammals, GLP-2 is liberated from proglucagon in the intestine and brain but not in pancreas, as a result of cell-specific expression of prohormone convertases in gut endocrine cells (Dhanvantari et al., Mol. Endocrinol. 10: 342-355 (1996); Rothenberg et al., Mol. Endocrinol. 10: 334-341 (1996); Damholt et al., Endocrinology 140: 4800-4808 (1999); Holst, Trends Endocrinol Metab. 10: 229-235 (1999)). Analysis of rat and human plasma using a combination of high-performance liquid chromatography and site-specific GLP-2 antisera reveals the presence of two principal circulating molecular forms, GLP-2¹⁻³³ and GLP-2³⁻³³ (Hartmann *et al.,* Supra; Brubaker et al., Endocrinol. 138: 4837-4843 (1997); Hartmann et al., J. Clin. Endocrinol. Metab. 85: 2884-2888 (2000)). GLP-2¹⁻³³ is cleaved *in vivo* by the protease dipeptidyl peptidase IV (DPP IV), which removes the first two residues, histidine and alanine (HA). The resulting peptide GLP-2³⁻³³ is essentially inactive.

GLP-2 regulates gastric motility, gastric acid secretion, intestinal hexose transport, and increases the barrier function of the gut epithelium (reviewed in Drucker, J. Clin. Endocr. Metab. 86: 1759-1764 (2001)). It significantly enhances the surface area of the mucosal epithelium via stimulation of crypt cell proliferation and inhibition of apoptosis in the enterocyte and crypt compartments. (Drucker et al., Proc. Natl. Acad. Sci. U.S.A. 93: 7911-7916 (1996)). GLP-2 reduces mortality and decreases mucosal injury, cytokine expression, and bacterial septicemia in small and large bowel inflammation (Boushey et al., Am. J. Physiol. 277: E937-E947 (1999); Prasad et al., J. Pediatr. Surg. 35: 357-359 (2000)). GLP-2 also enhances nutrient absorption and gut adaptation in rodents or humans with short bowel syndrome (SBS) (Jeppesen et al., Gastroenterology 120: 806-815 (2001)).

The actions of GLP-2 are transduced by the GLP-2 receptor (GLP-2R), a G protein-coupled receptor expressed in gut endocrine cells of the stomach, small bowel, colon, as well as enteric neurons and subendothelial myofibroblasts (Munroe et al., Proc. Natl. Acad. Sci U.S.A. 96: 1569-1573 (1999); Yusta et al., Gastroenterology 119: 744-755 (2000); Bjerknes et al., Proc. Natl. Acad. Sci. U.S.A. 98: 12497-12502 (2001); Orskov et al., Regul. Pept. 124: 105-112 (2005)). Direct activation of GLP-2R signaling in transfected baby hamster kidney fibroblasts expressing the GLP-2 receptor (BHK-GLP-2R cells) confers resistance to cycloheximide-induced apoptosis (Yusta et al., J. Biol. Chem. 275: 35345-35352 (2000)).

The cytoprotective, reparative, and energy-retentive properties of GLP-2 suggest that GLP-2 may potentially be useful for the treatment of human disorders characterized by injury and/or dysfunction of the intestinal mucosal epithelium. Intestinal epithelial injury is seen in patients with inflammatory bowel disease (IBD), including Crohn's Disease and ulcerative colitis, and in patients with autoimmune diseases that are associated with an inflammatory response in the intestine, such as Celiac's Disease (reviewed in Hanauer, New England J. Med. 334: 841-848 (1996)). In addition, some chemotherapy drugs cause injury to the intestinal epithelium that result in toxic side effects that are dose limiting (Oster, Oncology 13: 41 (1999)). Increased intestinal permeability is also reported in cases of acute pancreatitis (Kouris et al., Am. J. Surg. 181: 571-575 (2001)) and could contribute to food allergies by allowing macromolecules to access the subendothelial compartment (Troncone et al., Allergy 49: 142-146 (1994)).

As an important regulatory hormone in nutrient absorption, GLP-2 is also promising in treating patients with short bowel syndrome (SBS) (Drucker *et al*., *Supra;* Rubin, Gastroenterol. 117:261-263 (1999); Nightingale, Gut 45: 478-479 (1999)). SBS is defined as malabsorption resulting from anatomical or functional loss of a significant length of the small intestine (reviewed in Jeppesen, J. Nutr. 133: 3721-3724 (2003)). The causes of short bowel syndrome differ between adults and children: in adults, it most often results after surgery for Crohn's disease or mesenteric infarction; while in infants, the causes more commonly include necrotizing enterocolitis, gastroschisis, atresia, and volvulus (Platell et al., World J. Gastroenterol. 8: 13-20 (2002)).

Teduglutide, a DPP-IV resistant GLP-2 peptide analog (where alanine-2 is substituted with glycine (A2G)), is being developed for the potential treatment of gastrointestinal (GI) diseases, including SBS, Crohn's disease and pediatric GI disorders. Teduglutide also has potential for the treatment of mucositis associated with cancer chemotherapy and IBD. However, due to the peptide's low molecular weight, teduglutide is cleared quickly with a half-life of less than 30 minutes. Accordingly, daily dosing is required to maintain the therapeutic level (Shin et al., Curr. Opin. Endocrin. Diabetes 12: 63-71 (2005)). US2001/021767 discloses the sequence of GLP-2 and some analogs. Therefore, a need exists for a modified GLP-2 that will overcome the short half-life while retaining its function and provide for facile development and manufacture. W02005/097175 discloses the mimetibody platform.

Inflammatory ileus, the temporary impairment of coordinated gastrointestinal motility following invasive surgery or traumatic injury, remains a major clinical problem, extending hospital stays and often contributing to medical complications during the recovery period (Holte and Kehlet, Br. J. Surg. 87: 1480-1493 (2000)). Ileus is characterized by delayed gastric emptying, dilatation of the small bowel and colon, abdominal distension, loss of normal propulsive contractile patterns, and inability to evacuate gas or stool, leading to prolonged patient discomfort (abdominal distension, nausea, emesis).

In susceptible individuals, such as the elderly or patients with cardiopulmonary compromise, ileus can lead to more serious complications including acute gastric dilatation, cardiac arrhythmia, respiratory distress, aspiration pneumonia, and failure of surgical anastomoses. In severe cases, prolonged loss of the normal "housekeeping" contractile activity of the GI tract can contribute to bacterial overgrowth and breakdown of intestinal barrier function, followed by bacterial translocation and entry into the systemic circulation (Anup and Balasubramanian, J. Surg. Res. 92: 291-300 (2000)). This in turn can lead to endotoxemia, sepsis, multi-organ failure and ultimately death, an outcome for which elderly patients are the most susceptible. Even in the absence of complications, the return of normal bowel function is a prime limiting factor for release of patients from hospital, with inflammatory ileus increasing hospital stays by 3 to 5 days. Thus, costs accrued from increased morbidity and protracted hospital stays can be substantial.

Factors that contribute to the development and maintenance of ileus include the activation of central sympathetic inhibitory reflexes which release norepinephrine into the bowel wall, inhibitory humoral agents, anesthetic and analgesic agents, and inflammatory mediators (Livingston and Passaro, Dig. Dis. Sci. 35: 121-132 (1990); Bauer et al., Curr. Opin. Crit. Care 8: 152-157 (2002)). Results from rodent studies suggest that inflammation within the wall of the GI tract plays a central role in initiating and maintaining ileus.

Studies employing rodent models of post-operative ileus demonstrate that the muscularis externa is a highly immunologically active compartment. Normally resident within the muscularis externa is an impressive array of common leukocytes (Mikkelsen, Histol. Histopathol. 10: 719-736 (1995); Kalff et al., Ann. Surg. 228: 652-663 (1998)). Most abundant of these are resident macrophages, which form an extensive network of cells from the esophagus to the colon, and which are poised to defend the gastrointestinal tract from potential injury and disese. Disturbances to the bowel during abdominal surgery activate this macrophage network, initiating a local molecular inflammatory response. The activated macrophages release pro-inflammatory cytokines (IL-6, IL-1β, TNFα) and chemokines (MCP-1) that suppress neuromuscular communication within the muscularis and induce the expression of adhesion molecules (ICAM-1, P-selectin) on the vascular endothelium (Kalff et al., J. Leukoc. Biol. 63: 683-691 (1998); Josephs et al., J. Surg. Res. 86: 50-54 (1999); Kalff et al., Gastroenterology 117: 378-387 (1999); Kalff et al., Gastroenterology 118: 316-327 (2000); Wehner et al., Surgery 137: 436-46 (2005)). This in turn leads to a cellular inflammatory response characterized by recruitment of leukocytes (monocytes, neutrophils, T-cells, mast cells) from the systemic circulation, where there is a positive correlation between the magnitude of the inflammatory cell infiltrate and the severity of ileus (Kalff et al., Surgery 126: 498-509 (1999)). Infiltrating leukocytes release additional cytokines as well as prostaglandins, nitric oxide, proteases and reactive oxygen species that further contribute to neuromuscular dysfunction (von Ritter et al., Gastroenterology 97: 605-609 (1989); Bielefeldt and Conklin, Dig. Dis. Sci. 42: 878-884 (1997)).

To date, there are few options available in the clinic for management of inflammatory ileus. Prokinetics such as cisapride and neostigmine have been shown to improve postoperative bowel motility (Shibata and Toyoda, Surg. Today 28: 787-791 (1998)). However, results are inconsistent and these drugs have an increased risk of adverse cardiovascular effects that have proven difficult to predict in terms of severity and patient susceptibility. COX-2 inhibitors have been shown in animal studies to be protective against postoperative dysmotility of the small bowel(Schwarz et al., Gastroenterology 121: 1354-1371 (2001)), but had little effect on colonic dysmotility (Turler et al., Anal. Surg., 231(1): 56-66 (2002)). Phase I clinical trials in humans were completed comparing celecoxib and rofecoxib (Bouras et al., Neurogastroenterol. Motil. 16: 729-735 (2004)), and neither agent was found to improve post-operative motility.

The rapid return to oral feeding after surgery has been promoted as a means to stimulate normal hormonal regulation of motility patterns. This was found to hasten the return of bowel function and to improve comfort in a subset of patients when used as part of a multi-modal approach to bowel rehabilitation (Holte & Kehlet, Minerva. Anestesiol., 68(4): 152-156 (2002)). However, this treatment did not result in a significant reduction in the length of hospital stay. Furthermore, adequate stimulation of hormonal patterns requires a threshold caloric load that many patients are unable to tolerate.

One of the most common factors contributing to the development of prolonged ileus is the administration of opioid analgesics for postoperative pain relief. Opioids exert their analgesic effects by interacting with one or more of three receptor subtypes present on neurons in the pain processing centers of the brain. Most current opioid analgesics, such as morphine, work primarily by activating µ-(mu) and δ(delta)-opioid receptors. However, these same receptors are also expressed on the neurons within the gastrointestinal tract that control bowel motility. Activation of the receptors, whether in the presence or absence of inflammatory ileus, significantly suppresses gastrointestinal contractile function, causing bowel stasis and constipation. Adalor Corporation has conducted Phase I and II clinical trials using Alvimopan, a peripherally restricted and selective p-OR antagonist that does not cross the blood-brain barrier. When given in conjunction with opioid analgesics, Alvimopan prevented opioid-induced suppression of intestinal motility (Gonenne et al., Clin. Gastroenterol. Hepatol. 3: 784-791 (2005)). When compared with placebo, Alvimopan was found to hasten return of bowel function and to shorten hospital stay in patients who were experiencing mild to moderate postoperative ileus after having undergone abdominal surgery (Viscusi et al., Surg. Endosc. 20: 64-70 (2006)). Alvimopan does not alter inflammation.

To date, there are no safe and reliable treatment options available for the treatment of inflammatory ileus. The most effective remedies currently available are supportive in nature or ameliorate the compounding effects of opioid analgesia. They do not address inflammation as the underlying cause of ileus. Therefore, a significant unmet medical need remains.

### Brief Description of the Drawings

Fig. 1 shows the image of a SDS-PAGE gel of GLP-2 mimetibody Pro substitution variants (SED ID NOs: 43 and 44) after incubation with U937 cell lysate.
Fig. 2 shows a dose-dependent increase of the wet weight of mucosal scrapings from mice treated with A2G GLP-2 mimetibody.
Fig. 3 shows a significant acceleration of intestinal transit in mice treated with A2G-GLP-2 peptide. Statistical significance determined by unpaired Student's T-test.
Fig. 4 shows a significant acceleration of intestinal transit in mice treated with A2G GLP-2 mimetibody. Statistical significance determined by unpaired Student's t-test.
Fig. 5 shows significant attenuation of the delay in gastrointestinal transit associated with post-operative inflammatory ileus in mice treated with murine A2G GLP-2 mimetibody. Statistical significance determined by ANOVA followed by Bonferroni post hoc test.
Fig. 6 shows the whole mounts of intestinal muscularis with increased numbers of myeloperoxidase (MPO)-containing leukocytes following abdominal surgery. Treatment with murine A2G GLP-2 mimetibody significantly reduced the number of infiltrating cells, whereas IgG2a had no effect.
Fig. 7 shows the histogram with compiled cell counts from Fig. 6. Statistical significance determined by ANOVA followed by Bonferroni post hoc test.

### Summary of the Invention

One aspect of the invention is a mimetibody having the generic formula (II):

(GLP2RAg-Lk-V2-Hg-C_{H}2-C_{H}3)₍ₜ₎ (II)

where GLP2RAg is a mammalian GLP-2R agonist, Lk is a polypeptide linkage longer than 20 residues, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain C_{H}3 constant region and t is independently an integer from 1 to 10.

Another aspect of the invention is a mimetibody comprising a polypeptide having the sequence shown in SEQ ID NO: 10.

A mimetibody comprising a polypeptide having the sequence shown in SEQ ID NO: 4, 5, 6, 7, 8, 9, 11, 42, 43, 44, 45, 58, 59, 60, 61, 62, 63, 64, 65, 75, or 77 is disclosed herein.

A polynucleotide comprising a polynucleotide having the sequence shown in SEQ ID NO: 12, 13, 14, 15, 16, 17, 18, 46, 47, 48, 49, 66, 67, 68, 69, 70, 71, 72, 73, 76, or 78 or a complementary sequence is disclosed herein.

Another aspect of the invention is a polynucleotude comprising a polynucleotide encoding the amino acid sequence shown in SEQ ID NO: 10.

A polynucleotide comprising a polynucleotide encoding the amino acid sequence shown in SEQ ID NO: 4, 5, 6, 7, 8, 9, 11, 42, 43, 44, 45, 58, 59, 60, 61, 62, 63, 64, 65, 75, or 77 is dislosed herein.

A polypeptide comprising a polypeptide having the sequence shown in SEQ ID NO: 52, 54, 55 or 74 is disclosed herein.

A polynucleotide comprising a polynucleotide encoding the amino acid sequence shown in SEQ ID NO: 52, 54, 55, or 74 is disclosed herein.

Another aspect of the invention is a GLP-2 mimetibody for use in a method of reducing the symptoms of, or treating a disorder characterized by injury and/or dysfunction of the intestinal mucosal epithelium.

Another aspect of the invention is a GLP-2 mimetibody for use in a method of preventing, reducing the symptoms of, or treating inflammatory ileus.

### Detailed Description of the Invention

Single letter amino acid codes are used herein as understood by those skilled in the art. Numbering of amino acid residues in immunoglobulin constant regions is based on residue one being the N-terminal amino acid in a wild type IgG1 or IgG4 Fc domain.

The present invention provides protein constructs having the properties and activities of mammalian GLP-2. One embodiment of the invention is protein constructs that mimic different types of immunoglobulin molecules such as IgA, IgD, IgE, IgG, or IgM, and any subclass thereof, such as IgA1, IgA2, IgG1, IgG2, IgG3 or IgG4, or combinations thereof, hereinafter referred to as "GZP-2-mimetibodies" or simply "mimetibodies." Another embodiment of the invention is polypeptides that are variants of GLP-2 where the polypeptides have the properties and activities of the wild type molecule. The invention also provides nucleic acids encoding GLP-2 mimetibodies, polypeptides, vectors containing these nucleic acids, host cells, compositions and methods of making and using GLP-2 mimetibodies and polypeptides.

### GLP-2 Mimetibodies, polypeptides and compositions

The present invention generally relates to mimetibody polypeptides having the generic formula (I):

(Pep-Lk-V2-Hg-C_{H}2-C_{H}3)₍ₜ₎ (I)

where Pep is a polypeptide having a desired biological property, Lk is a polypeptide linkage longer than 20 residues, V2 is a portion of a C-terminus of an immunoglobulin variable region, Hg is at least a portion of an immunoglobulin hinge region, C_{H}2 is an immunoglobulin heavy chain C_{H}2 constant region and C_{H}3 is an immunoglobulin heavy chain C_{H}3 constant region and t is independently an integer of 1 to 10.

More particularly, the present invention relates to GLP-2 mimetibody polypeptides that are capable of, upon binding, activating GLP-2R. The polypeptides have the generic formula (II):

(GLP2RAg-Lk-V2-Hg-C_{H}2-C_{H}3) ₍ₜ₎ (II)

where GLP2RAg is a mammalian GLP-2R agonist, Lk is a polypeptide linkage longer than 20 residues, V2 is a portion of a C-terminus of an immunoglobulin variable region, Hg is at least a portion of an immunoglobulin hinge region, C_{H}2 is an immunoglobulin heavy chain C_{H}2 constant region and C_{H}3 is an immunoglobulin heavy chain C_{H}3 constant region and t is independently an integer of 1 to 10.

As used herein, "GLP-2R agonist" encompasses any molecule which, upon binding to, activates GLP-2R. GLP-2R agonists include wild-type mammalian GLP-2 and peptidic analogs of GLP-2. An exemplary wild-type GLP-2 peptide has the amino acid sequence shown in SEQ ID NO: 1. It is known that certain amino acid residues in naturally occurring GLP-2 can be substituted for other amino acid residues with the analogs maintaining the GLP-2R binding property of the wild-type GLP-2. For example, Ala2 of the wild-type human GLP-2 peptide can be substituted with Ser (A2S) or Gly (A2G). The resulting amino acid sequences are shown in SEQ ID NOs: 2 and 3, respectively.

In the present invention, novel analogs of wild-type human GLP-2 have been developed that function as GLP-2R agonists. Amino acid sequences of these analogs are shown in SEQ ID NOs: 50, 51, 52, 53, 54, 55, 56, 57, and 74 shown below (mutations designated against wild type GLP-2). These analogs are useful as GLP2RAg components of a mimetibody.

| SEQ ID NO: | Amino acid sequence | Mutations |
|---|---|---|
| 50 | HGDGSFSSDMSTILDNLAARDFINWLIQTKITD | A2G; D8S, E9D, N11S |
| 51 | HGDGSFSSDVSTILDNLAARDFINWLIQTKITD | A2G, D8S, E9D, M10V, N11S |
| 52 | HGDGSFSDEMNTYLDNLAARDFINWLIQTKITD | A2G, I13Y |
| 53 | HGDGSFSDEMNTILDGLAARDFINWLIQTKITD | A2G, N16G |
| 54 | HGDGSFSDEMNTILDNQAARDFINWLIQTKITD | A2G, L17Q |
| 55 | HGDGSFSDEMNTILDGQAARDFINWLIQTKITD | A2G, N16G, L17Q |
| 56 | HGDGSFSDEMNTILDNLAARDFIAWLIQTKITD | A2G, N24A |
| 57 | HGDGSFSDEMNTILDNLAARDFINWLVKGKITD | A2G, I27V, Q28K, T29G |
| 74 | HGDGSFSDEVNTILDNLAARDFINWLIQTKITD | A2G, M10V |

It has been observed that GLP-2 peptides can self-associate and pose a problem for development and manufacture of a homogeneous therapeutic candidate. See US Patent Application Publication No. 20040122210 A1. As described in the Examples below, the polypeptides having the amino acid sequences shown in SEQ ID NOs: 52, 54, 55 and 74 were designed to be monomeric at pH 7.5 and have reduced helical propensities. Accordingly, these human GLP-2 peptide analogs would be particularly useful in a mimetibody construct or as a naked therapeutic peptide.

In the mimetibodies disclosed herein, the linker portion (Lk) provides structural flexibility by allowing the mimetibody to have alternative orientations and binding properties. Exemplary linkers include non-peptide chemical linkages or one to 20 amino acids linked by peptide bonds, wherein the amino acids are selected from the 20 naturally occurring amino acids. The linker portion can include a majority of amino acids that are sterically unhindered, such as glycine, alanine and serine and include GS, GGGS (SEQ ID NO: 19), GSGGGS (SEQ ID NO: 20), and polymers or combinations thereof. Exemplary linkers within the scope of the invention may be longer than 20 residues and may include residues other than glycine, alanine and serine.

In the mimetibodies of the invention, V2 is a portion of a C-terminal domain of an immunoglobulin variable region such as a heavy chain variable region. An exemplary V2 amino acid sequence is GTLVTVSS (SEQ ID NO: 21).

It has been shown that O-glycosylation can occur at the two Tyr residues in the V2 region, although the extent of glycosylation is highly dependent on the host cell line and may also be influenced by culture conditions. O-glycans may act to block aggregation and proteolysis, resulting in greater *in vivo* stability. However, it may be desirable to abrogate the O-glycosylation because of heterogeneity and poor reproducibility. Accordingly, an alternative exemplary V2 amino acid sequence is GALVAVSS (SEQ ID NO: 22).

In the mimetibodies of the invention, Hg is a portion of the hinge domain of an immunoglobulin variable region such as a heavy chain variable region. Exemplary Hg amino acid sequences include EPKSCDKTHTCPPCP (SEQ ID NO: 23), EPKSADKTHTCPPCP (SEQ ID NO: 24), ESKYGPPCPSCP (SEQ ID NO: 25), ESKYGPPCPPCP (SEQ ID NO: 26) and CPPCP (SEQ ID NO: 27).

In the mimetibodies of the invention, C_{H}2 is an immunoglobulin heavy chain C_{H}2 constant region. Exemplary C_{H}2 amino acid sequences include: and

In the mimetibodies of the invention, C_{H}3 is an immunoglobulin heavy chain C_{H}3 constant region. Exemplary C_{H}3 amino acid sequences include: and It will be recognized by those skilled in the art that the C_{H}3 region of the mimetibodies of the invention may have its C-terminal amino acid cleaved off when expressed in certain recombinant systems.

In the mimetibodies of the invention, the FcRn scavenger receptor binding site of the immunoglobulin molecules is preserved at the junction of the C_{H}2 and C_{H}3 region. Since FcRn binding enables the return of pinocytosed immunoglobulin back to the extracellular space, it is expected that the half-life of GLP-2 mimetibodies will be significantly extended relative to GLP-2 peptides.

In one embodiment of the mimetibodies of the invention, the monomeric structure (GLP2-Lk-V2-Hg-C_{H}2-C_{H}3) can be linked to other monomers non-covalently or by covalent linkage, such as, but not limited to, a Cys-Cys disulfide bond.

IgG1 and IgG4 subclasses differ in the number of cysteines in the hinge region. Like the IgG1 subclass, there are two cysteines in the IgG4 hinge that participate in the disulfide bonding between heavy chains. However, the cysteine in IgG1 hinge that is normally involved in disulfide bonding to the light chain is absent in the IgG4 hinge. Therefore, the IgG4 hinge is less flexible than the IgG1 hinge.

In addition, the two isotypes differ in their ability to mediate complement dependent cytotoxicity (CDC) and antibody-dependent cellular cytotoxicity (ADCC). CDC is the lysing of a target in the presence of complement. The complement activation pathway is initiated by the binding of the first component of the complement system (C1q) to a molecule complexed with a cognate antigen. IgG1 is a strong inducer of the complement cascade and subsequent CDC activity, while IgG4 has little complement-inducing activity.

ADCC is a cell-mediated reaction in which nonspecific cytotoxic cells that express Fc receptors (FcRs) (*e.g*., Natural Killer (NK) cells, neutrophils, and macrophages) recognize bound antibody on a target cell and subsequently cause lysis of the target cell. The IgG1 subclass binds with high affinity to the Fc receptor and contributes to ADCC while IgG4 binds only weakly. The relative inability of IgG4 to activate effector functions is desirable since delivery of the mimetibody to cells without cell killing is possible.

Furthermore, the binding site for the FcRn scavenger receptor is present in IgG4 and IgG1 isotypes and both have similar binding characteristics. Therefore, the pharmacokinetics of the IgG1 and IgG4 mimetibodies of the invention are expected to be similar.

The hinge-C_{H}2-C_{H}3 portion of the immunoglobulin region (Hg-C_{H}2- C_{H}3) may also be extensively modified to form variants in accordance with the invention. For example, one or more native sites that provide structural features or functional activity not required by the mimetibody molecules could be removed. These sites may be removed by, for example, substituting or deleting residues, inserting residues into the site, or truncating portions containing the site. Exemplary Hg-C_{H}2-C_{H}3 variants are discussed below.
1. Sites involved in disulfide bond formation can be removed by deletion or substitution with other amino acids in the mimetibodies of the invention. Typically, the cysteine residues present in these motifs are removed or substituted. Removal of these sites may avoid disulfide bonding with other cysteine-containing proteins present in the mimetibody-producing host cell or intra-heavy chain disulfide bonding in IgG4-based constructs while still allowing for a dimeric CH3-CH2-hinge domain that is held together non-covalently.
   Most IgG type antibodies, such as IgG1, are homodimeric molecules made up of two identical heavy (H) chains and two identical light (L) chains, typically abbreviated H₂L₂. Thus, these molecules are generally bivalent with respect to antigen binding, i.e., both antigen binding (Fab) arms of the IgG molecule have identical binding specificity.
   IgG4 isotype heavy chains contain a CPSC (SEQ ID NO: 34) motif in their hinge regions capable of forming either inter-or intra-heavy chain disulfide bonds, i.e., the two Cys residues in the CPSC motif may disulfide bond with the corresponding Cys residues in the other H chain (inter) or the two Cys residues within a given CPSC motif may disulfide bond with each other (intra). It is believed that *in vivo* isomerase enzymes are capable of converting inter-heavy chain bonds of IgG4 molecules to intra-heavy chain bonds and vice *versa* (Aalberse and Schuurman, Immunology 105, 9-19 (2002)). Accordingly, since the HL pairs in those IgG4 molecules with intra-heavy chain bonds in the hinge region are not covalently associated with each other, they may dissociate into HL monomers that then reassociate with HL monomers derived from other IgG4 molecules forming bispecific, heterodimeric IgG4 molecules. In a bispecific IgG antibody the two Fabs of the antibody molecule differ in the epitopes that they bind. Substituting Ser228 in the hinge region of IgG4 with Pro results in "IgG1-like behavior," *i.e*., the molecules form stable disulfide bonds between heavy chains and therefore, are not susceptible to HL exchange with other IgG4 molecules.
2. The H-C_{H}2-C_{H}3 can be modified to make the mimetibodies of the invention more compatible with a selected host cell. For example, when a mimetibody of the invention is expressed recombinantly in a bacterial cell such as *E. coli*, the Pro-Ala sequence in the hinge may be removed to prevent digestion by the *E coli* enzyme proline iminopeptidase.
3. A portion of the hinge region can be deleted or substituted with other amino acids in the mimetibodies of the invention to prevent heterogeneity in the products expressed in a selected host cell.
4. One or more glycosylation sites can be removed in the mimetibodies of the invention. Residues that are typically glycosylated (*e.g*., Asn) may confer an Fc-dependent, cell-mediated cytolytic activity to the mimetibody. Such residues may be deleted or substituted with residues that are not glycosylated such as Ala.
5. Sites involved in interaction with complement, such as the Clq binding site, are removed in the mimetibodies of the invention.
6. Sites can be removed that affect binding to Fc receptors other than an FcRn salvage receptor in the mimetibodies of the invention. For example, the Fc receptors involved in ADCC activity can be removed in the mimetibodies of the invention. For example, mutation of Leu234/Leu235 in the hinge region of IgG1 to L234A/L235A or Phe234/Leu235 in the hinge region of IgG4 to P234A/L235A minimizes FcR binding and reduces the ability of the immunoglobulin to mediate complement dependent cytotoxicity and ADCC.

One embodiment of the present invention is a GLP-2 mimetibody according to formula (II) where the Hg-C_{H}2-C_{H}3 is from the IgG4 subclass and contains a Ser228Pro (S228P) substitution and P234A/L235A mutations. The complete polypeptide sequences of exemplary GLP-2 mimetibodies having these mutations and A2S and A2G in GLP-2 peptide sequence are shown respectively in SEQ ID NOs: 4 and 5. These sequences contain all of the domains of the mimetibody construct, namely the GLP2RAg-Lk-V2-Hg-C_{H}2-C_{H}3 domains. These mimetibody constructs are expected to be a homogeneous and stable population that does not trigger FcR-mediated effector functions. The substitution and mutations shown here are exemplary; Hg-C_{H}2-C_{H}3 domains within the scope of this invention may include other substitutions, mutations and/or deletions.

The partial polypeptide sequences of an exemplary A2G based GLP-2 mimetibody of the invention is shown in SEQ ID NO: 10.

The partial polypeptide sequences of other exemplary A2G based GLP-2 mimetibodies disclosed herein with variable linker lengths are shown in SEQ ID NOs: 6, 7, 8, 9 and 11. These sequences show all domains with the exception of the C_{H}2 and C_{H}3 domains. It will be understood by those skilled in the art that a C_{H}2 and C_{H}3 domain would be contained in a functional mimetibody.

The partial polypeptide sequences of other exemplary GLP-2 mimetibodies disclosed herein based on the GLP-2 analogs having the amino acid sequences shown in SEQ ID NOs: 50, 51, 52, 53, 54, 55, 56, and 57 are shown in SEQ ID NOs: 58, 59, 60, 61, 62, 63, 64, and 65, respectively. These sequences show all domains with the exception of the C_{H}2 and C_{H}3 domains. It will be understood by those skilled in the art that a C_{H}2 and C_{H}3 domain would be contained in a functional mimetibody.

The present invention includes GLP-2 mimetibodies that are capable of, upon binding, activating GLP-2R. The mimetibodies of the present invention can bind GLP-2R with a wide range of affinities. The affinity of a GLP-2 mimetibody for GLP-2R can be determined experimentally using any suitable method, for example, methods using Biacore or KinExA instrumentation, ELISA and competitive binding assays.

The GLP-2 mimetibodies and polypeptides of the present invention are useful in treating disorders or symptoms characterized by inflammation, injury and/or dysfunction of the intestinal mucosal epithelium. Effects of GLP-2 are also noted in bone formation and maintenance, and in central nervous system mediated disorders due to its role as a central satiety factor. Diseases or symptoms that can be treated using GLP-2 mimetibodies or polypeptides of the invention include, but are not limited to, GI diseases, including SBS, inflammatory bowel disease (IBD), Crohn's disease, colitis, pancreatitis, ileitis, inflammatory ileus (both postoperative and from other causes), mucositis associated with cancer chemotherapy and/or radiotherapy, intestinal atrophy caused by total parenteral nutrition or ischemia, bone related disorders such as osteoporosis, nutrient related disorders including obesity, and pediatric GI disorders including intestinal failure due to necrotizing enterocolitis in newborn infants. GLP-2 mimetibodies or polypeptides of the present invention can also be used to prevent, reduce the symptoms of, and treat inflammatory ileus.

Accordingly, another aspect of the present invention is pharmaceutical compositions comprising at least one GLP-2 mimetibody or polypeptide of the invention and a pharmaceutically acceptable carrier or diluent known in the art. The carrier or diluent can be a solution, suspension, emulsion, colloid or powder.

A GLP-2 mimetibody or polypeptide of the invention is formulated as a pharmaceutical composition in a therapeutically or prophylactically effective amount. The term "effective amount" generally refers to the quantities of mimetibody or polypeptide necessary for effective therapy, i.e., the partial or complete alleviation of the symptom or disorder for which treatment was sought. Included within the definition of effective therapy are prophylactic treatments intended to reduce the likelihood of onset of the above-described symptoms or disorders.

The composition can optionally comprise at least one further compound, protein or composition useful for treating the disease states discussed herein. For example, the mimetibodies or polypeptides of the invention can be used in combination with glutamine or other nutritional supplements are contemplated to increase body weight, aid in intestinal healing or improve nutrient absorbsion. Further, combination with anti-inflammatory agents are also contemplated. The term "in combination with" as used herein and in the claims means that the described agents can be administered to a mammal together in a mixture, concurrently as single agents or sequentially as single agents in any order.

### Nucleic acids, vectors and cell lines

Another aspect of the present invention is isolated nucleic acid molecules comprising, complementary to or having significant identity with a polynucleotide encoding at least one GLP-2 mimetibody or polypeptide of the invention. Other aspects of the present invention include recombinant vectors comprising at least one isolated GLP-2 mimetibody or polypeptide of the invention encoding nucleic acid molecule and cell lines and organisms that are capable of expressing the nucleic acid molecules. The nucleic acids, expression vectors and cell lines may generally be used to produce the mimetibody of the invention.

In one embodiment, the nucleic acid composition of the invention encodes a polypeptide having an amino acid sequence identical to or substantially homologous to SEQ ID NO: 10.

In one embodiment, the nucleic acid compositions disclosed herein encode polypeptides having amino acid sequences identical to or substantially homologous to any one of SEQ ID NOs: 4, 5, 6, 7, 8, 9, 11, 42, 43, 44, 45, 58, 59, 60, 61, 62, 63, 64, 65, 74, 75, and 77. Exemplary nucleic acid sequences that encode the polypeptide sequences shown in SEQ ID NO: 5, 6, 7, 8, 9, 10, 11, 42, 43, 44, 45, 58, 59, 60, 61, 62, 63, 64, 65, 75, and 77 are shown in SEQ ID NO: 12, 13, 14, 15, 16, 17, 18, 46, 47, 48, 49, 66, 67, 68, 69, 70, 71, 72, 73, 76, and 78, respectively. Also disclosed are allelic variations of the above-described nucleic acids.

Typically, the nucleic acids of the present invention are used in expression vectors for the preparation of the GLP-2 mimetibody or polypeptides of the invention. Vectors within the scope of the invention provide necessary elements for eukaryotic expression, including viral promoter driven vectors, such as CMV promoter driven vectors, *e.g*., pcDNA3.1, pCEP4 and their derivatives, Baculovirus expression vectors, Drosophila expression vectors and expression vectors that are driven by mammalian gene promoters, such as human Ig gene promoters. Other examples include prokaryotic expression vectors, such as T7 promoter driven vectors, *e.g*., pET41, lactose promoter driven vectors and arabinose gene promoter driven vectors.

The present invention also relates to cell lines expressing GLP-2 mimetibodies or polypeptides of the invention. The host cells can be prokaryotic or eukaryotic cells. Exemplary eukaryotic cells are mammalian cells, such as but not limited to, COS-1, COS-7, HEK293, BHK21, CHO, BSC-1, HepG2, 653, SP2/0, NS0, 293, HeLa, myeloma, lymphoma cells, or any derivative thereof. Most preferably, the host cells are HEK293, NS0, SP2/O or CHO cells. The cell lines of the present invention may stably express at least one GLP-2 mimetibody. The cell lines may be generated by stable or transient transfection procedures that are well known in the art.

The present invention further provides methods for expressing at least one GLP-2 mimetibody or polypeptide comprising culturing the cell lines under conditions wherein the GLP-2 mimetibody or polypeptide is expressed in detectable or recoverable amounts. The present invention also provides methods for generating at least one GLP-2 mimetibody or polypeptide comprising translating the GLP-2 mimetibody or polypeptide encoding nucleic acids under conditions *in vitro* or *in situ*, such that the GLP-2 mimetibody or polypeptide is expressed in detectable or recoverable amounts. The present invention also encompasses GLP-2 mimetibodies or polypeptides produced by the above methods.

A GLP-2 mimetibody can be recovered and purified by well-known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylatpatite chromatography and lectin chromatography. Reversed phase high performance liquid chroatography (RP-HPLC) can also be employed for purification.

Alternatively, a GLP-2 derived polypeptide of the invention can be prepared by chemical synthesis techniques well known to those skilled in the art. Polypeptides of the invention produced by either recombinant or chemical methods can be recovered and purified by methods well known to those skilled in the art.

### Methods of use

The GLP-2 mimetibodies or polypeptides are useful as, *inter alia,* research reagents and therapeutic agents. In one aspect, the present invention relates to their use in a method of modifying the biological activities of GLP-2 comprising providing at least one GLP-2 mimetibody or polypeptide to a mammal in need thereof. The GLP-2 mimetibody or polypeptide may activate cell signaling cascades through GLP-2R. In particular, the GLP-2 mimetibody or polypeptide may function as an agonist of GLP-2R. The term "agonist" is used in the broadest sense and includes a molecule that is capable of, directly, partially or fully activating, increasing or promoting one or more biological activities of GLP-2R.

The present invention also provides GLP-2 mimetibodies for use in methods for reducing the symptoms of, or treating at least one GLP-2 related condition or disease comprising administering a therapeutically effective amount of at least one GLP-2 mimetibody or polypeptide pharmaceutical composition to a patient in need thereof. The conditions and diseases suitable for treatment using the methods of the present invention include but are not limited to GI diseases, including SBS, Crohn's disease, and pediatric GI disorders, mucositis associated with cancer chemotherapy, IBD, inflammatory ileus, and other diseases and conditions described above.

GLP-2 interacts preferentially with GLP-2R found primarily on neurons of the enteric nervous system, and on GLP-2 containing enteroendocrine cells (Guan et al., Gastroenterology 130: 150-164 (2006)). One of the primary functions of GLP-2 is to promote columnar cell proliferation in the villus crypts where it enhances epithelial cell turnover and mucosal wound healing (Bulut et al., Regul. Pept. 121: 137-43 (2004)), enhances mucosal barrier function (Benjamin et al., Gut 47: 112-119 (2000)), and inhibits cell death by apoptosis (Brubaker and Drucker, Endocrinology 145: 2653-2659 (2004)). These effects have been shown to be neurally dependent as GLP-2R is not expressed in crypt columnar epithelial cells (Bjerknes and Cheng, Proc. Natl. Acad. Sci. U.S.A. 98: 12497-12502 (2001)). The presence of GLP-2R on enteric neurons suggests that GLP-2 may modify motility as well as neuro-immune interactions that play a role in intestinal inflammation.

Accordingly, the present invention further provides GLP-2 mimetibodies for use in methods of preventing, reducing the symptoms of, or treating inflammatory ileus, comprising administering a GLP-2 polypeptide composition or a GLP-2 mimetibody composition to a patient in need thereof. As used herein, "inflammatory ileus" can be ileus of any portion of the gastrointestinal tract, e.g., the stomach, small intestine and/or the colon. In addition, "inflammatory ileus" can result from any factor that causes ileus, e.g., surgery, including abdominal surgery such as transplantation surgery or abdominal surgery other than transplantation surgery, bowel surgery such as bowel resection, and orthopaedic surgery; traumatic injury, e.g., falls, car accident, personal assault, or any sequelae resulting from traumatic injury, e.g. limb fractures, rib fractures, fractures of the spine, thoracic lesions, ischaemia, retroperitoneal haematoma; intraperitoneal inflammation, e.g., intraabdominal sepsis, acute appendicitis, cholecystitis, pancreatitis, ureteric colic, basal pneumonia; myocardial infarction; metabolic disturbances; or any combination thereof.

As described above, the GLP-2 mimetibody or polypeptide pharmaceutical composition comprises an effective amount of at least one GLP-2 mimetibody or polypeptide and a pharmaceutically acceptable carrier or diluent. The effective amount for a given therapy, whether curative or preventative, will generally depend upon may different factors, including means of administration, target site and other medicants administered. Thus, treatment doses will need to be titrated to optimize safety and efficacy.

The methods of the present invention can optionally further comprise co-administration or combination therapies with any standard therapy used to treat the diseases listed above.

The mode of administration can be any suitable route to deliver the pharmaceutically effective amount of GLP-2 mimetibody or polypeptide of the present invention to a host. For example, the GLP-2 mimetibody or polypeptide can be delivered via parenteral administration, such as subcutaneous, intramuscular, intradermal, intravenous or intranasal administration, or any other means known in the art.

The present invention is further described with reference to the following examples. These examples are merely to illustrate aspects of the present invention and are not intended as limitations of this invention.

### Example 1

### Cloning, Expression and Purification of GLP-2 mimetibodies in Mammalian Cells

Nucleic acid sequence encoding A2S GLP-2 was generated in a 2-step PCR amplification. The first round amplification was performed using forward primer 5'-CCAAAGTATACAGGCGCATAGCGATGGTTCTTTCTCTGATGAGATGAACACCATTCTTG-3' (SEQ ID NO: 37) and reverse primer 5'-TTGGTCTGAATCAACCAGTTTATAAAGTCTCGAGCGGCAAGATTATCAAGAATGGTGTTCA TCTC-3' (SEQ ID NO: 38). The melting, annealing and extension temperature were set at 96°C, 48°C, and 72°C, respectively. Three cycles of reactions were carried out.

For the second round amplification, the forward primer included a NotI restriction enzyme recognition site and the reverse primers included a BamHI site. The sequence of the forward primer is 5'-TTTGCGGCCGCCCAAAGTATACAGGCG-3' (SEQ ID NO: 39) and reverse primer 5'-AAAGGATCCGTCAGTGATTTTGGTCTGAATCAACCAG-3' (SEQ ID NO: 40). The melting, annealing and extension temperature were set at 96°C, 48°C, and 60°C, respectively. Thirty cycles of reactions were carried out.

Nucleic acid sequence encoding A2G GLP-2 was generated in the same procedure except the forward primer used in the first round of amplification is 5'-CCAAAGTATACAGGCGCATGGCGATGGTTCTTTCTCTGATGAGATGAACACCATTCTTG-3' (SEQ ID NO: 41).

The amplified PCR products (A2S and A2G GLP-2) were cloned into the NotI/BamHI sites of a CMV promoter driven, human IgG4 ΔCH1, Ser to Pro, Ala/Ala expression vector using standard cloning procedures.

The A2S and A2G GLP-2 IgG4 mimetibodies were transiently expressed in HEK 293E cells and purified from the conditioned media using protein A affinity chromatography according to standard procedures. The eluted material from the protein A affinity column was further subjected to a size exclusion column for further purification.

The purified A2S and A2G GLP-2 mimetibodies were analyzed by SDS-polyacrylamide gel electrophoresis (SDS-PAGE) and size exclusion chromatography coupled to static light scattering analysis (SEC-SLS). The migration of the purified mimetibodies on SDS-PAGE under both reducing and non-reducing denaturing conditions was in the expected range. Analysis by SEC-SLS showed a protein with a molecular weight of approximately 123KD corresponding to the dimer of the mimetibodies. Since the GLP-2 Mimetibodies migrate as a monomer on the SDS-PAGE gel, the dimerization is via noncovalent interactions.

### Example 2

### cAMP Expression Assay

In order to evaluate the *in vitro* activities of the GLP-2 mimetibodies, a cAMP expression assay was developed. To achieve this goal, a clonal cell line expressing a mutated human GLP-2R was generated by transfecting HEK 293E cells. The mutated human GLP-2R differs from the wild-type human GLP-2R (SEQ ID NO: 35) at three amino acid positions within the C-terminal intracellular region (SEQ ID NO: 36). GLP-2 peptide stimulated cAMP expression in this cell line and the stimulation was specific, as a control peptide did not stimulate cAMP expression.

A2S and A2G IgG4 GLP-2 mimetibodies were compared with the corresponding GLP-2 peptides (A2S and A2G) for their ability to stimulate cAMP expression in the recombinant cell line. Briefly, cells were incubated with individual GLP-2 mimetibody or GLP-2 peptide for 30 minutes. The cAMP expression was quantitated using the cAMP Direct Screen System (Cat. No. CSD 200, Applied Biosystems, Bedford, MA). The EC₅₀ for A2S and A2G peptides are 0.5nM and 0.8nM, respectively; the EC₅₀ for A2S and A2G mimetibodies are 2.2nM and 3.8nM, respectively. Therefore, the potency of the GLP-2 mimetibodies in this assay was -4-fold less than the peptide.

### Example 3

### GLP-2 Mimetibody Variants

To investigate the effect of linker length on the GLP-2 mimetibody, different constructs with various linker lengths were generated. The sequences of the core region are shown below in Table 1.

These variants were expressed transiently in HEK 293 cells, purified and analyzed by SDS-PAGE. Analysis by SEC-SLS showed a peak with a molecular weight of 65-70 kDa corresponding to the monomer of the mimetibodies in addition to one corresponding to the dimer of the mimetibodies. It was observed that the longer the linker length, the higher the proportion of the monomer population.

Linker length and V2 region variants were tested in the cAMP expression assay described in Example 2. The data demonstrated that the activities of the GLP-2 mimetibodies, as measured by EC₅₀, directly correlated with the linker length, i.e., mimetibodies with longer linkers have higher activity (Table 1).

**Table 1. Core amino acid sequences and EC₅₀ of GLP-2 mimetibodies with variable linker length.**

| SEQ ID NO: | Amino acid sequence | EC₅₀ (nM) |
|---|---|---|
| 5* | HGDGSFSDEMNTILDNLAARDFINWLIQTKITDGSGGGSGTLVTVSSESKYGPPCPPCP | 5.1 |
| 6 | HGDGSFSDEMNTILDNLAARDFINWLIQTKITDGGGGSCPPCP | 90 |
| 7 | HGDGSFSDEMNTILDNLAAPDFINWLIQTKITDGGGGSGGGGSCPPCP | 22.5 |
| 8 | HGDGSFSDEMNTILDNLAARDFINWLIQTKITDGGGGSGGGGSGGGGSCPPCP | 12.7 |
| 9 | HGDGSFSDEMNTILDNLAARDFINWLIQTKITDGGGGSGGGGSGGGGSGGGGSCPPCP | 4.6 |
| 10 | | 2.1 |
| 11** | HGDGSFSDEMNTILDNLAARDFINWLIQTKITDGSGGGSGALVAVSSESLYGPPCPPCP | 3.1 |

| | | |
|---|---|---|
| * Only amino acid numbers 1-59 Of SEQ ID NO: 5 is shown in the table. ** SEQ ID NO: 11 shows a V2 region variant. | | |

In order to increase the stability of GLP-2 mimetibodies, a series of variants were constructed in which the amino acid residues at the proteolytic cleavage sensitive sites in the V2 or Hg region were substituted with Pro. The core region sequences of the GLP-2 mimetibody variants are shown below in Table 2.

**Table 2. Core amino acid sequences and EC₅₀ of GLP-2 mimetibodies with Pro substitution.**

| SEQ ID NO: | Amino acid sequence | EC₅₀ (nM) |
|---|---|---|
| 42 | HGDGSFSDEMNTILDNLAARDFINWLIQTKITDGSGGGSGALVPVSSESKYGPPCPPCP | 3.6 |
| 43 | HGDGSFSDEMNTILDNLAARDFINWLIQTKITDGSGGGSGALVAVPSESKYGPPCPPCP | 4.8 |
| 44 | HGDGSFSDEMNTILDNLAARDFINWLIQTKITDGSGGGSGALVAVSPESKYGPPCPPCP | 7.7 |
| 45 | HGDGSFSDEMNTILDNLAARDFINWLIQTKITDGSGGGSGALVAVSSESKPGPPCPPCP | 5.8 |

The Pro substitution variants were expressed transiently in HEK 293 cells, purified and analyzed by SDS-PAGE. The data from the cAMP expression assay demonstrated that the activities of these GLP-2 mimetibody variants, as measured by EC₅₀, are comparable to that of the A2G GLP-2 mimetibody (SEQ ID NO: 5).

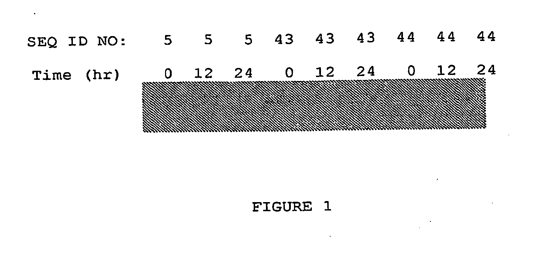

The purified Pro substitution variants were incubated with U937 cell lysate in the presence of CompleteMini protease inhibitor tablets (Cat. No. 1 836 153, Roche Applied Science, Indianapolis, IN) for 0, 12, or 24 hours. Afterwards, GLP-2 mimetibody variants were purified using Protein A beads and resolved on a SDS-PAGE gel. As shown in Figure 1, in comparison with the A2G GLP-2 mimetibody (SEQ ID NO: 5), there was less degradation in Pro substitution variants (SEQ ID NO: 43 or 44) in the 24 hour test period.

In conclusion, the Pro substitution variants are more resistant to proteolysis *in vitro.*

### Example 4

### GLP-2 Mimetibody Stimulates the Mucosal Weight Gain in Small Intestine

To demonstrate the *in vivo* activities of the GLP-2 mimetibodies, CD1 mice were injected with the GLP-2 mimetibodies and endpoints within the small intestine were evaluated. Briefly, female CD1 mice were given daily subcutaneous injections of A2G GLP-2 peptide (SEQ ID NO: 3), A2G GLP-2 IgG4 mimetibody (SEQ ID NO: 5), or control mimetibody for 10 days. Afterwards, the mice were euthanized and the small intestines were removed, flushed with saline, and processed as described below.

Specifically, 4cm sections were harvested: (1) immediately distal to the pylorus (duodenum), (2) starting 2cm distal of the ligament of Treitz (jejunum), and (3) immediately proximal to the cecum (ileum). The remaining small intestine from ∼6cm distal of the ligament of Treitz to 4cm proximal to the cecum was used to prepare mucosal scrapings. From the proximal and distal ends of the remnant, an equal distance was removed until a 15cm section remained, the remnant was cut longitudinally, rinsed, and the mucosal layer was removed using the short end of a glass microscope slide. The wet weight of the intact intestinal segments and mucosa was measured.

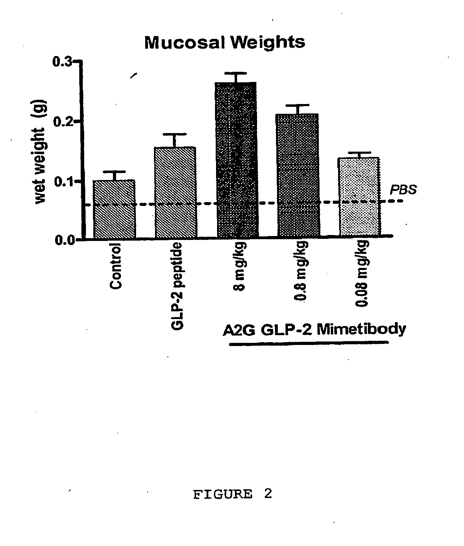

The weight of the mucosal scrapings taken from the 15cm segment between jejunum and ileum from different mice is shown in Figure 2. For mice injected with A2G GLP-2 mimetibody, a dose dependant increase in mucosal wet weight was observed. At 0.8 and 8 mg/kg (0.26 and 2.6 nmole, respectively), the increase was statistically significant comparing with the control mimetibody (p<0.0001 and p<0.0004, respectively).

A statistically significant increase (p<0.0001) was also seen in mice injected with the A2G GLP-2 peptide at 2.5 mg/kg (13.3 nmole). In comparison, the A2G GLP-2 mimetibody is effective *in vivo* at a 50-fold lower dose than the A2G GLP-2 peptide (on a molar basis).

### Example 5

### Pharmacokinetics of GLP-2 Mimetibody

To measure the pharmacokinetics of GLP-2 mimetibodies, CD1 mice were intravenously or subcutaneously dosed with 3mg/kg of the A2G GLP-2 mimetibody (SEQ ID NO: 5). Blood was collected at different time points into citrate buffer containing protease inhibitors to minimize the possibility of *ex vivo* degradation and plasma was separated by centrifugation.

A time resolved fluorescence (TRF) assay was used to measure active mimetibody. Active mimetibody reflects the intact N-terminus of the peptide still attached to the Fc region of the mimetibody.

Based on the TRF experiments, the calculated half-life of the A2G GLP-2 mimetibody in mice was 26.5 hours. In contrast, the reported half-life of GLP-2 peptide in humans is 7.2 +/- 2 minutes (Hartmann et al., J. Clin. Endocrinol. Metab. 85: 2884-2888 (2000)). Therefore, the half-life of A2G GLP-2 mimetibody is more than 200-fold higher than that of the GLP-2 peptide.

In a similar experiment, cynomolgus monkeys were intravenously dosed with 1mg/kg of the A2G GLP-2 mimetibody. Based on the TRF data, the calculated half-life of the A2G GLP-2 mimetibody in cynomolgus monkeys was 4.8 days.

### Example 6

### Pharmacodynamics of GLP-2 Mimetibody

Based on its extended pharmacokinetics, the GLP-2 mimetibody is expected to have a longer duration of response. To evaluate the pharmacodynamics of the A2G GLP-2 mimetibody, mice were dosed daily, every other day, weekly, or once only at the start of the study. To control for animal handling, on days that the mice did not receive A2G GLP-2 mimetibody, they were injected with the negative control mimetibody, *i.e.,* the mimetibody immunoglobulin scaffold without the GLP-2 peptide. The doses of the A2G GLP-2 mimetibody and the negative control were 4 mg/kg (1.3 nmoles/kg) for all groups. The duration of the study was 11 days and tissue was processed as described in Example 4.

Mice dosed with the A2G GLP-2 mimetibody once per week had a significantly increased mucosal weight compared to control mimetibody. The difference was more pronounced when the A2G GLP-2 mimetibody was administered every day, or every other day. Similar pattern was observed regarding the small intestinal section wet weight. In both the duodenum and jejunum, a significant increase in weight over the control mimetibody was seen with all regimens except for the single dose experiment.

### Example 7

### Mutations in GLP-2 Prevent Peptide Dimerization

Wild-type GLP-2 peptide (SEQ ID NO: 1) dimerizes at high concentration. For example, in PBS (pH 7.5), GLP-2 exists as a monomer at 0.4 mg/mL but as a mixture of monomers (about 20%) and reversibly self-associated dimer (about 80%) at 2 mg/mL (data not shown). The self-association poses a challenge to the development and manufacturing of a homogeneous therapeutic.

Peptide analogs (SEQ ID NOs: 52, 54, 55, and 74) were designed that retain wild-type GLP-2 biological activity and exist as a monomer at high concentration. GLP-2(A2G, L17Q) (SEQ ID NO: 54) and GLP-2(A2G, N16G, L17Q) (SEQ ID NO: 55) were synthesized and purified to >95% purity. Peptides GLP-2 (SEQ ID NO: 1), GLP-2(A2G) (SEQ ID NO: 3), and GLP-1 (SEQ ID NO: 79) were included as controls in the characterization of the analogs.

Solution molecular weight of the peptides was measured by SEC-SLS. Briefly, peptide solutions in PBS (pH 7.5) at 0.4 to 2.0 mg/mL were fractionated over a Superdex peptide column (Amersham Pharmacia). The eluted peaks were monitored by static light scattering at 690 nm and solution molecular weight was determined at UV 280 nm using the Astra software package (Wyatt Inc.).

At 1 mg/ml, GLP-1 eluted as a single peak with molecular weight within the expected monomer size. GLP-2 and GLP-2(A2G) displayed similar distributions of overlapping dimer and monomer peaks. Both analog peptides GLP-2(A2G, L17Q) and GLP-2(A2G, N16G, L17Q) eluted as single peaks with molecular weight consistent with mainly monomeric peptide.

The secondary structures of tested peptides were determined using 0.2 mg/mL peptide solutions in PBS. Briefly, CD spectra were collected in triplicate at 1 nm intervals at 25°C in 0.1 cm path length cell. Secondary structures were determined by fitting of the CD spectra using CD spectra software (CD Spectra Deconvolution software 2.1). All tested peptides contained peaks corresponding to the presence of alpha helices. However, helix content in the analog peptides GhP-2(A2G, L17Q) and GLP-2(A2G, N16G, L17Q) was -17%, similar to that of GLP-1, and lower than that of GLP-2 and GLP-2 (A2G) (Table 3).

**Table 3. Percentage of helical and random coil structure in GLP peptides**

| Structure | GLP-2 | GLP-2(A2G) | GLP-2 (A2G, L17Q) | GLP-2 (A2G, N16G, L17Q) | GLP-1 |
|---|---|---|---|---|---|
| Helix | 19.6% | 20.7% | 16.9% | 17.0% | 16.8% |
| Random coil | 37.9% | 37.1% | 41.8% | 41.4% | 41.9% |

In addition, trifluoroethanol (TFE) is known to induce helix formation in peptides (Soennichsen et al., Biochemistry 31: 8791 (1992)). Accordingly, helical propensity analysis using TFE was performed. Briefly, tested peptides were diluted to 0.2 mg/mL in PBS (pH 7.5) containing 0, 1, 5, 15, 33 or 50% TFE. CD spectra were collected and CD plots were generated after data averaging, buffer subtraction and curve smoothing. Helical propensity values were obtained from mean residue ellipticity (MRE) at 222 nm versus %TFE plots. The concentration of TFE that effected a 50% transformation of the CD spectrum at 222 nm was used as a measure of helical propensity.

The results showed that GLP-2 and GLP-2(A2G) displayed greater helical propensity, requiring ∼16% TFE. for transformation to maximum helix signal. In comparison, GLP-1 had lower helical propensity, requiring >20% TFE for helical transformation. Significantly, the analog peptides GLP-2(A2G, L17Q) and GLP-2(A2G, N16G, L17Q) both require >20% TFE for helical transformation, bearing a closer resemblance to GLP-1 than to GLP-2. Therefore, L17Q substitution decreased the helix-forming potential of the GLP-2 peptide.

### Example 8

### Mutations in GLP-2 Prevent Mimetibody Dimerization

Nucleic acid sequences encoding GLP-2 mimetibodies with A2G, L17Q (SEQ ID NO: 75) and A2G, N16G, L17Q (SEQ ID NO: 77) analogs were generated using the QuickChange XL kit from Stratagene. These mimetibody variants were transiently expressed in HEK 293E cells and purified following procedures described in Example 1.

Based on SEC-SLS analysis, GLP-2(A2G, N16G, L17Q) mimetibody exhibited molecular weight consistent with monomer while GLP-2(A2G, L17Q) mimetibody exhibited molecular weight reflective of a monomer and dimer mixture.

### Example 9

### In vitro Activity of GLP-2 Analog Mimetibodies

The *in vitro* activity of GLP-2 analogs was tested in a cAMP expression assay. This assay was based on the cAMP Direct Screen System from Applied Biosystems utilizing a cell line expressing mutated huGLP-2R in HEK 293E cells. Peptide at concentrations ranging from 0.01 nM to 1.0 uM in PBS with 0.5% BSA was added to -50,000 cells suspended in 96-well plates. After a 30-minute incubation at 37°C, Lysis Buffer followed by luminescence reagents (Applied Biosystems) was added according to the manufacturers' procedures (Applied Biosystems Luminescence protocol: cAMP-Screen Direct System). Luminescence was quantitated using a TopCount liquid scintillation analyzer (PerkinElmer), and data was processed using Softmax software (Molecular Devices Corporation). The EC-50 values obtained from plots of cAMP levels versus peptide concentration are listed in Table 5 below.

**Table 5: EC-50 values of GLP-2 peptides obtained from plots of cAMP versus peptide concentration.**

| Structures | Wt-GLP-2 | GLP-2_{(A2G)} | GLP-2_{(A2G, N16G, L17Q)} | GLP-2 _{(A2G, L17Q)} |
|---|---|---|---|---|
| cAMP in Vitro EC50 values (nM) | 1.6 | 1.9 | 3.5 | 5.2 |

The data indicate only 2x- and 3x-less activity of GLP-2_{(A2G,N16G, L17Q}) and GLP-2_{(A2G, L17Q)}, respectively, relative to wild type GLP-2.

### Example 10

### A2G-GLP-2 Peptide Accelerates Upper GI Transit

To test the effects of A2G-GLP-2 on upper gastrointestinal transit in normal mice, mice were randomly assigned to 2 groups (14 animals per test group). Each group received daily subcutaneous injection (total volume 200 ml) of either A2G-GLP-2 peptide (50 µg/mouse) or the phosphate-buffered saline vehicle for 10 consecutive days.

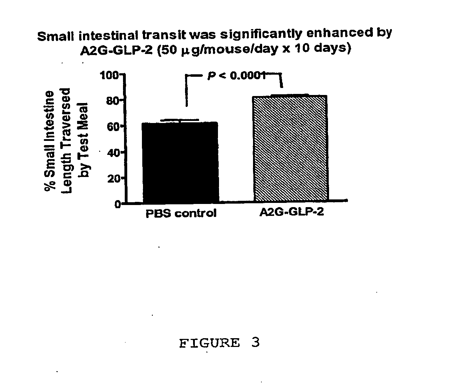

On the day of study, upper gastrointestinal transit was measured using the carmine dye technique. Mice were fed a test meal of 0.25 ml of a 6% (w/v) solution of carmine cochineal powder mixed into 0.5% (w/v) methylcellulose administered intragastrically by an 18 gauge curved feeding tube. Following oral test meal administration, mice were replaced into their home cages. Twenty minutes after marker meal administration, mice were rapidly euthanized by cervical dislocation and the entire gastrointestinal tract was excised starting from the distal colon and working towards the gastric pylorus. The resected gut was arranged lengthwise parallel to a linear metric scale ruler taking care to avoid stretching the organ. The linear distance traversed by the carmine dye front through the small intestine was measured together with the total length of the small bowel. Upper gastrointestinal transit was expressed as the percentage of the entire small intestine traversed by the carmine dye front during the 20-minute test period: % small intestine traveled = [distance traversed by dye front through small intestine (cm) / entire small intestine length (cm) x 100]. As shown in Fig. 3, A2G-GLP-2 treatment led to an acceleration of upper gastrointestinal transit.

### Example 11

### GLP-2 Mimetibody Accelerates Upper GI Transit

To test the effects of GLP-2 Mimetibody on upper gastrointestinal transit in normal mice, mice were randomly assigned to 2 groups (4 animals per group). Each group received a single injection of A2G GLP-2 mimetibody (SEQ ID NO: 5) (4 mg/kg) or the IgG4 negative control 4 days prior to measurement of gastrointestinal transit.

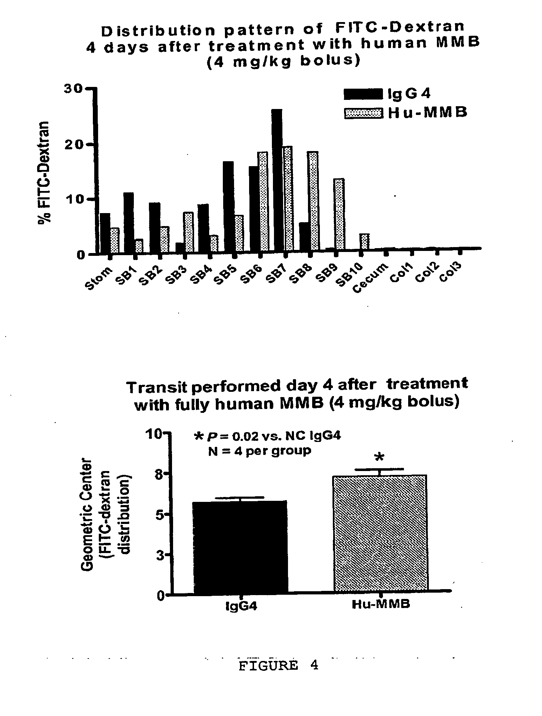

On the day of study, upper gastrointestinal transit was measured using the FITC-dextran method. This method provides both, a measure of gastrointestinal transit and a readout of the pattern of distribution of the test meal along the gastrointestinal tract. Mice were fed a test meal of 150 ml of FITC-dextran solution (5 mg/ml of 70,000 molecular weight dextran conjugated to fluorescein-isothiocyanate in 0.5% methylcellulose/deionized water) administered intragastrically by an 18 gauge curved feeding tube. Following oral administration of the FITC-dextran test meal, mice were returned to their home cages. It was shown that a 30 min test period was the optimum duration for detecting accelerated transit, while a 45 min test period was optimum for detecting delayed transit. Following the appropriate test period, mice were sacrificed by carbon dioxide exposure. The entire gastrointestinal tract from the lower esophageal sphincter to the terminal colon was removed. The bowel segments were opened along the mesenteric border. The tissue and luminal contents of the stomach, 10 equal segments of small intestine, the cecum, and 3 equal segments of colon were placed in individual Eppendorf tubes containing 1 ml of PBS. The tissue was vigorously mixed on a table-top vortex, and solid materials were pelleted by centrifugation.
Aliquots of the cleared supernatant were read in duplicate on a 96-well fluorescence plate reader to quantify the magnitude of the fluorescent signal in each segment of bowel. These values were used to calculate the Geometric Center (GC), which is defined as the weighted average distribution of the fluorescent signal along the gastrointestinal tract: GC = ∑ (% of total fluorescent signal per segment x segment number)/100. Higher values represent faster rates of transit on scale of 1 to 15. As shown in Fig. 4, one single does of GLP-2 mimetibody treatment led to an acceleration of upper gastrointestinal transit. The mimetibody-induced shift in the distribution pattern of FITC-dextran is shown in the upper panel. There is reduced labeling in the stomach, and an overall shift of the bulk of the label to more distal segments of the small bowel. The GC is calculated in the lower panel for statistical comparison. Normal mice exhibit a GC = 6 following a 30 minute test duration, and this was unchanged by treatment with IgG4 scaffold. Treatment with the GLP-2 mimetibody increased the GC to 7.5.

### Example 12

### GLP-2 Mimetibody Attenuates Impaired GI Motility Associated with Post-Operative Inflammatory Ileus

Due to immunogenicity of human IgG4 in mice, a murine GLP-2 mimetibody, i.e., human A2G-GLP2 peptide in murine IgG2a scaffold (SEQ ID NO: 80) was used in the following experiments.

To test the effects of GLP-2 mimetibody on impaired GI motility associated with post-operative inflammatory ileus, mice were randomized into 3 groups (8 animals per group) and treated with 2 mg/kg murine A2G-GLP-2 mimetibody, IgG2a or PBS. One hour later, the mice were subject to laparotomy and manipulation of the small intestine. Briefly, male CD-1 mice were anesthetized by inhaled isoflurane and prepared for surgery. The abdomen was shaved of hair and treated with antiseptic solution. The animal was then covered with a surgical drape. The abdomen was opened via mid-line laparotomy, and the entire small intestine was exteriorized onto the sterile drape. Using two moistened, sterile, cotton-tipped applicators, the small intestine was then gently compressed along its length from the ligament of Treitz to the ileo-cecal junction. The small intestine was then returned to the abdominal cavity and the incision sutured closed. Afterwards, the mice were returned to their home cages. A fourth group of 8 animals served as naive controls.

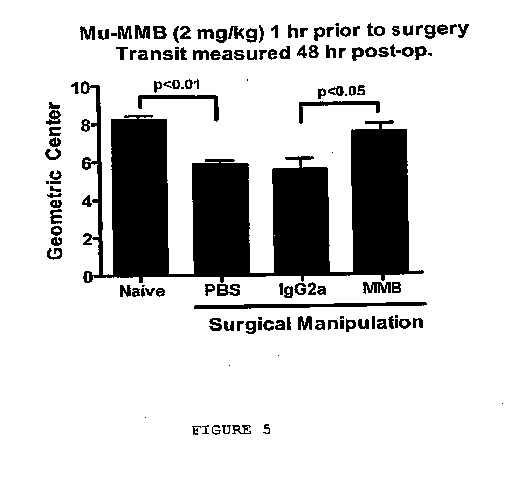

All mice received an oral test meal of FITC-dextran 48 hr after the operation. Gastrointestinal transit was determined 45 minutes after oral feeding. As shown in Fig. 5, naive controls exhibited a normal 45-minute transit (GC = 8.2). Abdominal surgery led to a significant delay in gastrointestinal transit in PBS-treated animals. Treatment with IgG2a had no effect on the surgically induced delay in transit, whereas treatment with murine A2G-GLP-2 mimetibody led to a significant improvement in transit.

### Example 13

### GLP-2 Mimetibody Reduces Cellular Inflammation Associated with Post-Operative Inflammatory Ileus

To test the effects of GLP-2 mimetibody on cellular inflammation, post-operative inflammatory ileus was induced in mice as described in Example 12. Myeloperoxidase histochemistry was performed on tissues harvested from the mid-small bowel of the mice 48 hr after the operation.

Briefly, segments of mid-small bowel were collected from the centrifuge tubes described in Example 12. Whole mounts of the muscle layer were prepared by pinning the tissue flat in a Sylgard^{®} lined Petri dish and stretching the tissue to 2 times its length and 1.5 times its width. The mucosa was removed by fine dissection. The muscularis whole mounts were then fixed with 100% ethanol for 1 hr, washed 3 times with PBS, and incubated for 20 min in PBS containing 0.1% hydrogen peroxide and 1 mg/ml Hanker-Yates reagent. Following a second wash with PBS, the whole mounts were mounted on glass slides, cover-slipped, and viewed on an optical microscope. Myeloperoxidase containing leukocytes were counted in 6 to 8 adjacent 200X optical fields of view and the mean cell counts were calculated and recorded.

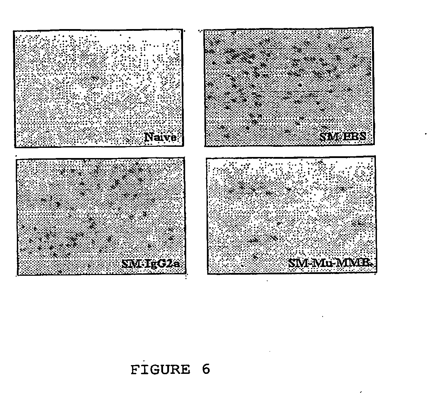

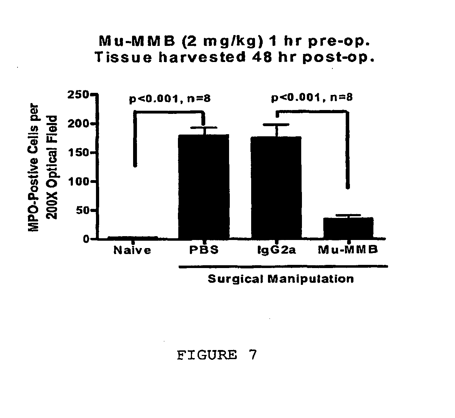

Representative whole mounts of intestinal muscularis stained for myeloperoxidase (MPO) activity using Hanker-Yates reagent are shown in Fig. 6. Black dots represent MPO-positive leukocytes infiltrating the small intestinal muscle layer. Few MPO-positive cells were found in tissue harvested from naive mice. A marked increase in the number of infiltrating leukocytes was found in mice treated with PBS prior to undergoing surgical manipulation of the small bowel. Treatment with IgG2a had no effect on the numbers of infiltrating cells. In contrast, treatment with murine A2G-GLP-2 mimetibody significantly reduced the number of infiltrating cells. Cell counts are compiled in Fig. 7 for statistical comparison.

## Claims

1. A mimetibody according to formula (II):
(GLP2RAg-Lk-V2-Hg-C_{H}2-C_{H}3) ₍ₜ₎ (II)
where GLP2RAg is a mammalian GLP-2R agonist which is wild-type mammalian GLP-2 or peptidic analogs of GLP-2, Lk is a polypeptide linkage longer than 20 residues, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain CH3 constant region and t is independently an integer from 1 to 10.

2. The mimetibody of claim 1 wherein GLP2RAg has the amino acid sequence of SEQ ID NO: 2, 3, 50, 51, 52, 53, 54, 55, 56, 57, or 74.

3. The mimetibody of claim 1 wherein Hg, C_{H}2 and C_{H}3 are of the IgG1 subclass.

4. The mimetibody of claim 3 wherein Cys220 of Hg is substituted with Ala, and Leu234 and Leu235 of CH2 are mutated to Ala234 and Ala235.

5. The mimetibody of claim 1 wherein Hg is of the IgG4 subclass, and CH2 and CH3 are of the IgG1 subclass.

6. The mimetibody of claim 1 wherein Hg, CH2 and CH3 are of the IgG4 subclass.

7. The mimetibody of claim 6 wherein Ser228 of Hg is substituted with Pro and Phe234 and Leu235 of CH2 are mutated to Ala234 and Ala235.

8. The mimetibody of claim 1 wherein the mimetibody binds to GLP-2 receptor.

9. The mimetibody of claim 1 comprising a polypeptide having the sequence shown in SEQ ID NO: 10.

10. A polynucleotide encoding a mimetibody according to any one of claims 1 to 9.

11. A vector comprising the polynucleotide of claim 10.

12. A cell line expressing a mimetibody according to any one of claims 1 to 9.

13. A cell line comprising the vector of claim 11.

14. The cell line of claim 13 wherein the cell line is HEK293, NSO, SP2/0 or CHO cells.

15. A method to produce a polypeptide comprising the steps of culturing the cell line of claim 14 and purifying the expressed polypeptide.

16. A pharmaceutical composition comprising an effective amount of at least one mimetibody according to any one of claims 1 to 9, and a pharmaceutically acceptable carrier or diluent.

17. The pharmaceutical composition of claim 16, for use in therapy.

18. The composition of claim 16 for use in (i) a method of reducing the symptoms of, or treating at least one GLP-2 related condition or disorder, wherein the GLP-2 related condition or disorder is a gastrointestinal disorder, a bone related disorder, or a nutrient related disorder, or (ii) preventing, reducing the symptoms of, or treating inflammatory ileus.

19. The composition of claim 16 for use according to claim 18, wherein the gastrointestinal disorder is short bowel syndrome, inflammatory bowel disease, Crohn's disease, colitis, pancreatitis, ileitis, mucositis, or intestinal atrophy.

20. The composition of claim 16 for use according to claim 18, wherein the gastrointestinal disorder is a pediatric gastrointestinal disorder.

21. The composition of claim 16 for use according to claim 18, wherein the bone related disorder is osteoporosis.

22. The composition of claim 16 for use according to claim 18, wherein the nutrient related disorder is obesity.

## Patentansprüche

1. Mimetikörper gemäß Formel (II) :
(GLP2RAg-Lk-V2-Hg-C_{H}2-C_{H}3) ₍ₜ₎ (II)
worin GLP2RAg für einen Säuger-GLP-2R-Agonisten, bei dem es sich um Wildtyp-Säuger-GLP-2 oder peptidische Analoge von GLP-2 handelt, Lk für eine Polypeptidverknüpfung mit einer Länge von mehr als 20 Resten, V2 für einen Anteil eines C-Terminus eines variablen Immunglobulinbereichs, Hg für wenigstens einen Anteil eines variablen Immunglobulin-Scharnierbereichs, C_{H}2 für einen konstanten C_{H}2-Bereich einer schweren Immunglobulinkette und C_{H}3 für einen konstanten C_{H}3-Bereich einer schweren Immunglobulinkette steht und t unabhängig eine ganze Zahl von 1 bis 10 bedeutet.

2. Mimetikörper nach Anspruch 1, wobei GLP2RAg die Aminosäuresequenz von SEQ ID NO: 2, 3, 50, 51, 52, 53, 54, 55, 56, 57 oder 74 aufweist.

3. Mimetikörper nach Anspruch 1, wobei Hg, C_{H}2 and C_{H}3 aus der IgG1-Subklasse stammen.

4. Mimetikörper nach Anspruch 3, wobei Cys220 von Hg mit Ala substituiert ist und Leu234 und Leu235 von CH2 zu Ala234 und Ala235 mutiert sind.

5. Mimetikörper nach Anspruch 1, wobei Hg aus der IgG4-Subklasse stammt und CH2 und CH3 aus der IgGl-Subklasse stammen.

6. Mimetikörper nach Anspruch 1, wobei Hg, CH2 und CH3 aus der IgG4-Subklasse stammen.

7. Mimetikörper nach Anspruch 6, wobei Ser228 von Hg mit Pro substituiert ist und Phe234 und Leu235 von CH2 zu Ala234 und Ala235 mutiert sind.

8. Mimetikörper nach Anspruch 1, wobei der Mimetikörper an GLP-2-Rezeptor bindet.

9. Mimetikörper nach Anspruch 1, umfassend ein Polypeptid mit der in SEQ ID NO: 10 dargestellten Sequenz.

10. Polynukleotid, codierend für einen Mimetikörper gemäß einem der Ansprüche 1 bis 9.

11. Vektor, umfassend das Polynukleotid nach Anspruch 10.

12. Zelllinie, exprimierend einen Mimetikörper gemäß einem der Ansprüche 1 bis 9.

13. Zelllinie, umfassend den Vektor nach Anspruch 11.

14. Zelllinie nach Anspruch 13, bei der es sich um HEK293-, NSO-, SP2/0- oder CHO-Zellen handelt.

15. Verfahren zur Herstellung eines Polypeptids, wobei man in den Verfahrensschritten die Zelllinie nach Anspruch 14 kultiviert und das exprimierte Polypeptid aufreinigt.

16. Pharmazeutische Zusammensetzung, umfassend eine wirksame Menge wenigstens eines Mimetikörpers gemäß einem der Ansprüche 1 bis 9 sowie ein pharmazeutisch unbedenkliches Träger- oder Verdünnungsmittel.

17. Pharmazeutische Zusammensetzung nach Anspruch 16, zur Verwendung in der Therapie.

18. Zusammensetzung nach Anspruch 16 zur Verwendung bei (i) einem Verfahren zur Verringerung der Symptome oder zur Behandlung wenigstens eines mit GLP-2 verbundenen Leidens bzw. wenigstens einer mit GLP-2 verbundenen Störung, bei dem bzw. der es sich um eine Magen-Darm-Erkrankung, eine mit Knochen verbundene Erkrankung oder eine mit Ernährung verbundene Erkrankung handelt, oder (ii) Vorbeugen, Verringern der Symptome oder Behandeln von entzündlichem Ileus.

19. Zusammensetzung nach Anspruch 16 zur Verwendung gemäß Anspruch 18, wobei es sich bei der Magen-Darm-Erkrankung um Kurzdarmsyndrom, entzündliche Darmkrankheit, Morbus Crohn, Colitis, Pankreatitis, Ileitis, Mucositis oder Darmatrophie handelt.

20. Zusammensetzung nach Anspruch 16 zur Verwendung gemäß Anspruch 18, wobei es sich bei der Magen-Darm-Erkrankung um eine Magen-Darm-Erkrankung bei Kindern handelt.

21. Zusammensetzung nach Anspruch 16 zur Verwendung gemäß Anspruch 18, wobei es sich bei der mit Knochen verbundenen Erkrankung um Osteoporose handelt.

22. Zusammensetzung nach Anspruch 16 zur Verwendung gemäß Anspruch 18, wobei es sich bei der mit Ernährung verbundenen Erkrankung um Fettleibigkeit handelt.

## Revendications

1. Miméticorps selon la formule (II) .
(GLP2RAg-Lk-V2-Hg-C_{H}2-C_{H}3)₍ₜ₎ (II)
où GLP2RAg est un agoniste de GLP-2R de mammifère qui est GLP-2 de mammifère de type sauvage ou des analogues peptidiques de GLP-2, Lk est un lieur polypeptidique d'une longueur supérieure à 20 résidus, V2 est une partie d'une extrémité C-terminale d'une région variable d'immunoglobuline, Hg est au moins une partie d'une région de charnière variable d'immunoglobuline, C_{H}2 est une région constante C_{H}2 de chaîne lourde d'immunoglobuline et C_{H}3 est une région constante C_{H}3 de chaîne lourde d'immunoglobuline et t est indépendamment un entier de 1 à 10.

2. Miméticorps de la revendication 1 dans lequel GLP2RAg a la séquence d'acides aminés de SEQ ID NO: 2, 3, 50, 51, 52, 53, 54, 55, 56, 57, or 74.

3. Miméticorps de la revendication 1 dans lequel Hg, C_{H}2 et C_{H}3 sont de la sous-classe IgG1.

4. Miméticorps de la revendication 3 dans lequel Cys220 de Hg est substitué par Ala, et Leu234 et Leu235 de CH2 sont mutés en Ala234 et Ala235.

5. Miméticorps de la revendication 1 dans lequel Hg est de la sous-classe IgG4, et CH2 et CH3 sont de la sous-classe IgG1.

6. Miméticorps de la revendication 1 dans lequel Hg, CH2 et CH3 sont de la sous-classe IgG4.

7. Miméticorps de la revendication 6 dans lequel Ser228 de Hg est substitué par Pro et Phe234 et Leu235 de CH2 sont mutés en Ala234 et Ala235.

8. Miméticorps de la revendication 1, le miméticorps se liant au récepteur GLP-2.

9. Miméticorps de la revendication 1 comprenant un polypeptide ayant la séquence décrite dans SEQ ID NO: 10.

10. Polynucléotide codant pour un miméticorps selon l'une quelconque des revendications 1 à 9.

11. Vecteur comprenant le polynucléotide de la revendication 10.

12. Lignée cellulaire exprimant un miméticorps selon l'une quelconque des revendications 1 à 9.

13. Lignée cellulaire comprenant le vecteur de la revendication 11.

14. Lignée cellulaire de la revendication 13, la lignée cellulaire étant les cellules HEK293, NSO, SP2/0 ou CHO.

15. Procédé pour produire un polypeptide comprenant les étapes de culture de la lignée cellulaire de la revendication 14 et purification du polypeptide exprimé.

16. Composition pharmaceutique comprenant une quantité efficace d'au moins un miméticorps selon l'une quelconque des revendications 1 à 9, et un véhicule ou diluant pharmaceutiquement acceptable.

17. Composition pharmaceutique de la revendication 16, pour utilisation en thérapie.

18. Composition de la revendication 16 pour utilisation dans (i) un procédé de réduction des symptômes de, ou de traitement d'au moins une affection ou un trouble associé à GLP-2, l'affection ou trouble associé à GLP-2 étant un trouble gastro-intestinal, un trouble osseux, ou un trouble associé à un nutriment, ou (ii) la prévention, la réduction des symptômes, ou le traitement d'un iléus inflammatoire.

19. Composition de la revendication 16 pour utilisation selon la revendication 18, le trouble gastro-intestinal étant le syndrome de l'intestin court, une maladie intestinale inflammatoire, la maladie de Crohn, la colite, la pancréatite, l'iléite, la mucite, ou l'atrophie intestinale.

20. Composition de la revendication 16 pour utilisation selon la revendication 18, le trouble gastro-intestinal étant un trouble gastro-intestinal pédiatrique.

21. Composition de la revendication 16 pour utilisation selon la revendication 18, le trouble osseux étant l'ostéoporose.

22. Composition de la revendication 16 pour utilisation selon la revendication 18, le trouble associé au nutriment étant l'obésité.


### SEQUENCE LISTING

<110> BAKER, AUDREY E. MOORE, BEVERLY A. NESSPOR, TOM O'NEIL, KARYN PALMER, JEFFREY M. PICHA, KRISTEN SAGUE, SARAH
<120> GLP-2 MIMETIBODIES, POLYPEPTIDES, COMPOSITIONS, METHODS AND USES
<130> CEN5115USNP
<140> TO BE ASSIGNED
   <141> 2006-10-24
<150> 60/729704
   <151> 2005-10-24
<150> 60/824160
   <151> 2006-08-31
<150> 60/862487
   <151> 2006-10-23
<160> 80
<170> FastSEQ for Windows Version 4.0
<210> 1
   <211> 33
   <212> PRT
   <213> Homo sapiens
<400> 1
   His Ala Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp
<210> 2
   <211> 33
   <212> PRT
   <213> Artificial Sequence
<220>
   <223> GLP-2 variant
<400> 2
   His Ser Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp
<210> 3
   <211> 33
   <212> PRT
   <213> Artificial Sequence
<220>
   <223> GLP-2 variant
<400> 3
   His Gly Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp
<210> 4
   <211> 275
   <212> PRT
   <213> Artificial Sequence
<220>
   <223> Mimetibody
<400> 4
   His Ser Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp Gly Ser Gly Gly Gly Ser Gly Thr Leu Val Thr Val Ser Ser Glu
   35 40 45
   Ser Lys Tyr Gly Pro Pro Cys Pro Pro Cys Pro Ala Pro Glu Ala Ala
   50 55 60
   Gly Gly Pro Ser Val Phe Leu Phe Pro Pro Lys Pro Asp Thr Leu Met
   65 70 75 80
   Ile Ser Arg Thr Pro Glu Val Thr Cys Val Val Val Asp Val Ser Gln
   85 90 95
   Glu Asp Pro Glu Val Gln Phe Asn Trp Tyr Val Asp Gly Val Glu Val
   100 105 110
   His Asn Ala Lys Thr Lys Pro Arg Glu Glu Gln Phe Asn Ser Thr Tyr
   115 120 125
   Arg Val Val Ser Val Leu Thr Val Leu His Gln Asp Trp Leu Asn Gly
   130 135 140
   Lys Glu Tyr Lys Cys Lys Val Ser Asn Lys Gly Leu Pro Ser Ser Ile
   145 150 155 160
   Glu Lys Thr Ile Ser Lys Ala Lys Gly Gln Pro Arg Glu Pro Gln Val
   165 170 175
   Tyr Thr Leu Pro Pro Ser Gln Glu Glu Met Thr Lys Asn Gln Val Ser
   180 185 190
   Leu Thr Cys Leu Val Lys Gly Phe Tyr Pro Ser Asp Ile Ala Val Glu
   195 200 205
   Trp Glu Ser Asn Gly Gln Pro Glu Asn Asn Tyr Lys Thr Thr Pro Pro
   210 215 220
   Val Leu Asp Ser Asp Gly Ser Phe Phe Leu Tyr Ser Arg Leu Thr Val
   225 230 235 240
   Asp Lys Ser Arg Trp Gln Glu Gly Asn Val Phe Ser Cys Ser Val Met
   245 250 255
   His Glu Ala Leu His Asn His Tyr Thr Gln Lys Ser Leu Ser Leu Ser
   260 265 270
   Leu Gly Lys
   275
<210> 5
   <211> 276
   <212> PRT
   <213> Artificial Sequence
<220>
   <223> Mimetibody
<400> 5
   His Gly Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp Gly Ser Gly Gly Gly Ser Gly Thr Leu Val Thr Val Ser Ser Glu
   35 40 45
   Ser Lys Tyr Gly Pro Pro Cys Pro Pro Cys Pro Ala Pro Glu Ala Ala
   50 55 60
   Gly Gly Pro Ser Val Phe Leu Phe Pro Pro Lys Pro Lys Asp Thr Leu
   65 70 75 80
   Met Ile Ser Arg Thr Pro Glu Val Thr Cys Val Val Val Asp Val Ser
   85 90 95
   Gln Glu Asp Pro Glu Val Gln Phe Asn Trp Tyr Val Asp Gly Val Glu
   100 105 110
   Val His Asn Ala Lys Thr Lys Pro Arg Glu Glu Gln Phe Asn Ser Thr
   115 120 125
   Tyr Arg Val Val Ser Val Leu Thr Val Leu His Gln Asp Trp Leu Asn
   130 135 140
   Gly Lys Glu Tyr Lys Cys Lys Val Ser Asn Lys Gly Leu Pro Ser Ser
   145 150 155 160
   Ile Glu Lys Thr Ile Ser Lys Ala Lys Gly Gln Pro Arg Glu Pro Gln
   165 170 175
   Val Tyr Thr Leu Pro Pro Ser Gln Glu Glu Met Thr Lys Asn Gln Val
   180 185 190
   Ser Leu Thr Cys Leu Val Lys Gly Phe Tyr Pro Ser Asp Ile Ala Val
   195 200 205
   Glu Trp Glu Ser Asn Gly Gln Pro Glu Asn Asn Tyr Lys Thr Thr Pro
   210 215 220
   Pro Val Leu Asp Ser Asp Gly Ser Phe Phe Leu Tyr Ser Arg Leu Thr
   225 230 235 240
   Val Asp Lys Ser Arg Trp Gln Glu Gly Asn Val Phe Ser Cys Ser Val
   245 250 255
   Met His Glu Ala Leu His Asn His Tyr Thr Gln Lys Ser Leu Ser Leu
   260 265 270
   Ser Leu Gly Lys
   275
<210> 6
   <211> 43
   <212> PRT
   <213> Artificial Sequence
<220>
   <223> Mimetibody
<400> 6
   His Gly Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp Gly Gly Gly Gly Ser Cys Pro Pro Cys Pro
   35 40
<210> 7
   <211> 48
   <212> PRT
   <213> Artificial Sequence
<220>
   <223> Mimetibody
<400> 7
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp Gly Gly Gly Gly Ser Gly Gly Gly Gly Ser Cys Pro Pro Cys Pro
           35                  40                  45
<210> 8
   <211> 53
   <212> PRT
   <213> Artificial Sequence
<220>
   <223> Mimetibody
<400> 8
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp Gly Gly Gly Gly Ser Gly Gly Gly Gly Ser Gly Gly Gly Gly Ser
           35                  40                  45
   Cys Pro Pro Cys Pro
       50
<210> 9
   <211> 58
   <212> PRT
   <213> Artificial Sequence
<220>
   <223> Mimetibody
<400> 9
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp Gly Gly Gly Gly Ser Gly Gly Gly Gly Ser Gly Gly Gly Gly Ser
           35                  40                  45
   Gly Gly Gly Gly Ser Cys Pro Pro Cys Pro
       50                  55
<210> 10
   <211> 63
   <212> PRT
   <213> Artificial Sequence
<220>
   <223> Mimetibody
<400> 10
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp Gly Gly Gly Gly Ser Gly Gly Gly Gly Ser Gly Gly Gly Gly Ser
           35                  40                  45
   Gly Gly Gly Gly Ser Gly Gly Gly Gly Ser Cys Pro Pro Cys Pro
       50                  55                  60
<210> 11
   <211> 59
   <212> PRT
   <213> Artificial Sequence
<220>
   <223> Mimetibody
<400> 11
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Ala Leu Val Ala Val Ser Ser Glu
           35                  40                  45
   Ser Lys Tyr Gly Pro Pro Cys Pro Pro Cys Pro
       50                  55
<210> 12
   <211> 831
   <212> DNA
   <213> Artificial Sequence
<220>
   <223> Mimetibody
<400> 12
   catggcgatg gttctttctc tgatgagatg aacaccattc ttgataatct tgccgctcga 60
   gactttataa actggttgat tcagaccaaa atcactgacg gatccggtgg aggctccggt 120
   accttagtca ccgtctcctc agagtccaaa tatggtcccc catgcccacc atgcccggcg 180
   cctgaggccg ccgggggacc atcagtcttc ctgttccccc caaaacccaa ggacactctc 240
   atgatctccc ggacccctga ggtcacgtgc gtggtggtgg acgtgagcca ggaagacccc 300
   gaggtccagt tcaactggta cgtggatggc gtggaggtgc ataatgccaa gacaaagccg 360
   cgggaggagc agttcaacag cacgtaccgt gtggtcagcg tcctcaccgt cctgcaccag 420
   gactggctga acggcaagga gtacaagtgc aaggtctcca acaaaggcct cccgtcctcc 480
   atcgagaaaa ccatctccaa agccaaaggg cagcctcgag agccacaggt gtacaccctg 540
   cccccatccc aggaggagat gaccaagaac caggtcagcc tgacctgcct ggtcaaaggc 600
   ttctacccca gcgacatcgc cgtggagtgg gagagcaatg ggcagccgga gaacaactac 660
   aagaccacgc ctcccgtgct ggactccgac ggctccttct tcctctacag caggctaacc 720
   gtggacaaga gcaggtggca ggaggggaat gtcttctcat gctccgtgat gcatgaggct 780
   ctgcacaacc actacacaca gaaaagcttg tccctgtctc tgggtaaatg a          831
<210> 13
   <211> 129
   <212> DNA
   <213> Artificial Sequence
<220>
   <223> Mimetibody
<400> 13
   catggcgatg gttctttctc tgatgagatg aacaccattc ttgataatct tgccgctcga 60
   gactttataa actggttgat tcagaccaaa atcactgacg gaggaggtgg atcctgccca 120
   ccatgcccg                                                          129
<210> 14
   <211> 144
   <212> DNA
   <213> Artificial Sequence
<220>
   <223> Mimetibody
<400> 14
   catggcgatg gttctttctc tgatgagatg aacaccattc ttgataatct tgccgctcga 60
   gactttataa actggttgat tcagaccaaa atcactgacg gaggaggtgg atccggtggt 120
   ggcggcagtt gcccaccatg cccg                                         144
<210> 15
   <211> 159
   <212> DNA
   <213> Artificial Sequence
<220>
   <223> Mimetibody
<400> 15
   catggcgatg gttctttctc tgatgagatg aacaccattc ttgataatct tgccgctcga 60
   gactttataa actggttgat tcagaccaaa atcactgacg gaggaggtgg atccggcggt 120
   ggcggatctg gtggtggcgg cagttgccca ccatgcccg                         159
<210> 16
   <211> 174
   <212> DNA
   <213> Artificial Sequence
<220>
   <223> Mimetibody
<400> 16
   catggcgatg gttctttctc tgatgagatg aacaccattc ttgataatct tgccgctcga 60
   gactttataa actggttgat tcagaccaaa atcactgacg gaggaggtgg atccggtgga 120
   ggaggctcag gcggtggcgg atctggtggt ggcggcagtt gcccaccatg cccg        174
<210> 17
   <211> 189
   <212> DNA
   <213> Artificial Sequence
<220>
   <223> Mimetibody
<400> 17
   catggcgatg gttctttctc tgatgagatg aacaccattc ttgataatct tgccgctcga 60
   gactttataa actggttgat tcagaccaaa atcactgacg gaggaggtgg atccggcgga 120
   ggaggttccg gtggaggagg ctcaggcggt ggcggatctg gtggtggcgg cagttgccca 180
   ccatgcccg                                                         189
<210> 18
   <211> 177
   <212> DNA
   <213> Artificial Sequence
<220>
   <223> Mimetibody
<400> 18
   catggcgatg gttctttctc tgatgagatg aacaccattc ttgataatct tgccgctcga 60
   gactttataa actggttgat tcagaccaaa atcactgacg gatccggtgg aggctccggt 120
   gccttagtcg ccgtctcctc agagtccaaa tatggtcccc catgcccacc atgcccg    177
<210> 19
   <211> 4
   <212> PRT
   <213> Artificial Sequence
<220>
   <223> Linker
<400> 19
   Gly Gly Gly Ser
   1
<210> 20
   <211> 6
   <212> PRT
   <213> Artificial Sequence
<220>
   <223> Linker
<400> 20
   Gly Ser Gly Gly Gly Ser
   1               5
<210> 21
   <211> 8
   <212> PRT
   <213> Homo sapiens
<400> 21
   Gly Thr Leu Val Thr Val Ser Ser
   1               5
<210> 22
   <211> 8
   <212> PRT
   <213> Artificial Sequence
<220>
   <223> V2 variant
<400> 22
   Gly Ala Leu Val Ala Val Ser Ser
   1 5
<210> 23
   <211> 15
   <212> PRT
   <213> Homo sapiens
<400> 23
   Glu Pro Lys Ser Cys Asp Lys Thr His Thr Cys Pro Pro Cys Pro
   1 5 10 15
<210> 24
   <211> 15
   <212> PRT
   <213> Homo sapiens
<400> 24
   Glu Pro Lys Ser Ala Asp Lys Thr His Thr Cys Pro Pro Cys Pro
   1 5 10 15
<210> 25
   <211> 12
   <212> PRT
   <213> Homo sapiens
<400> 25
   Glu Ser Lys Tyr Gly Pro Pro Cys Pro Ser Cys Pro
   1 5 10
<210> 26
   <211> 12
   <212> PRT
   <213> Homo sapiens
<400> 26
   Glu Ser Lys Tyr Gly Pro Pro Cys Pro Pro Cys Pro
   1 5 10
<210> 27
   <211> 5
   <212> PRT
   <213> Homo sapiens
<400> 27
   Cys Pro Pro Cys Pro
   1 5
<210> 28
   <211> 110
   <212> PRT
   <213> Homo sapiens
<400> 28
   Ala Pro Glu Leu Leu Gly Gly Pro Ser Val Phe Leu Phe Pro Pro Lys
   1 5 10 15
   Pro Lys Asp Thr Leu Met Ile Ser Arg Thr Pro Glu Val Thr Cys Val
   20 25 30
   Val Val Asp Val Ser His Glu Asp Pro Glu Val Lys Phe Asn Trp Tyr
   35 40 45
   Val Asp Gly Val Glu Val His Asn Ala Lys Thr Lys Pro Arg Glu Glu
   50 55 60
   Gln Tyr Asn Ser Thr Tyr Arg Val Val Ser Val Leu Thr Val Leu His
   65 70 75 80
   Gln Asp Trp Leu Asn Gly Lys Glu Tyr Lys Cys Lys Val Ser Asn Lys
   85 90 95
   Ala Leu Pro Ala Pro Ile Glu Lys Thr Ile Ser Lys Ala Lys
   100 105 110
<210> 29
   <211> 110
   <212> PRT
   <213> Homo sapiens
<400> 29
   Ala Pro Glu Ala Ala Gly Gly Pro Ser Val Phe Leu Phe Pro Pro Lys
   1 5 10 15
   Pro Lys Asp Thr Leu Met Ile Ser Arg Thr Pro Glu Val Thr Cys Val
   20 25 30
   Val Val Asp Val Ser His Glu Asp Pro Glu Val Lys Phe Asn Trp Tyr
   35 40 45
   Val Asp Gly Val Glu Val His Asn Ala Lys Thr Lys Pro Arg Glu Glu
   50 55 60
   Gln Tyr Asn Ser Thr Tyr Arg Val Val Ser Val Leu Thr Val Leu His
   65 70 75 80
   Gln Asp Trp Leu Asn Gly Lys Glu Tyr Lys Cys Lys Val Ser Asn Lys
   85 90 95
   Ala Leu Pro Ala Pro Ile Glu Lys Thr Ile Ser Lys Ala Lys
   100 105 110
<210> 30
   <211> 110
   <212> PRT
   <213> Homo sapiens
<400> 30
   Ala Pro Glu Phe Leu Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 31
   <211> 110
   <212> PRT
   <213> Homo sapiens
<400> 31
   Ala Pro Glu Ala Ala Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 32
   <211> 107
   <212> PRT
   <213> Homo sapiens
<400> 32
   Gly Gln Pro Arg Glu Pro Gln Val Tyr Thr Leu Pro Pro Ser Arg Asp
    1               5                  10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 33
   <211> 107
   <212> PRT
   <213> Homo sapiens
<400> 33
   Gly Gln Pro Arg Glu Pro Gln Val Tyr Thr Leu Pro Pro Ser Gln Glu
   1               5                   10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Arg Leu Thr Val Asp Lys Ser Arg Trp Gln Glu Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Leu Gly Lys
               100                 105
<210> 34
   <211> 4
   <212> PRT
   <213> Homo sapiens
<400> 34
   Cys Pro Ser Cys
   1
<210> 35
   <211> 553
   <212> PRT
   <213> Homo sapiens
<400> 35
   Met Lys Leu Gly Ser Ser Arg Ala Gly Pro Gly Arg Gly Ser Ala Gly
   1               5                   10                  15
   Leu Leu Pro Gly Val His Glu Leu Pro Met Gly Ile Pro Ala Pro Trp
               20                  25                  30
   Gly Thr Ser Pro Leu Ser Phe His Arg Lys Cys Ser Leu Trp Ala Pro
           35                  40                  45
   Gly Arg Pro Phe Leu Thr Leu Val Leu Leu Val Ser Ile Lys Gln Val
       50                  55                  60
   Thr Gly Ser Leu Leu Glu Glu Thr Thr Arg Lys Trp Ala Gln Tyr Lys
   65                  70                  75                  80
   Gln Ala Cys Leu Arg Asp Leu Leu Lys Glu Pro Ser Gly Ile Phe Cys
                   85                  90                  95
   Asn Gly Thr Phe Asp Gln Tyr Val Cys Trp Pro His Ser Ser Pro Gly
               100                 105                 110
   Asn Val Ser Val Pro Cys Pro Ser Tyr Leu Pro Trp Trp Ser Glu Glu
           115                 120                 125
   Ser Ser Gly Arg Ala Tyr Arg His Cys Leu Ala Gln Gly Thr Trp Gln
       130                 135                 140
   Thr Ile Glu Asn Ala Thr Asp Ile Trp Gln Asp Asp Ser Glu Cys Ser
   145                 150                 155                 160
   Glu Asn His Ser Phe Lys Gln Asn Val Asp Arg Tyr Ala Leu Leu Ser
                   165                 170                 175
   Thr Leu Gln Leu Met Tyr Thr Val Gly Tyr Ser Phe Ser Leu Ile Ser
               180                 185                 190
   Leu Phe Leu Ala Leu Thr Leu Leu Leu Phe Leu Arg Lys Leu His Cys
           195                 200                 205
   Thr Arg Asn Tyr Ile His Met Asn Leu Phe Ala Ser Phe Ile Leu Arg
       210                 215                 220
   Thr Leu Ala Val Leu Val Lys Asp Val Val Phe Tyr Asn Ser Tyr Ser
   225                 230                 235                 240
   Lys Arg Pro Asp Asn Glu Asn Gly Trp Met Ser Tyr Leu Ser Glu Met
                   245                 250                 255
   Ser Thr Ser Cys Arg Ser Val Gln Val Leu Leu His Tyr Phe Val Gly
               260                 265                 270
   Ala Asn Tyr Leu Trp Leu Leu Val Glu Gly Leu Tyr Leu His Thr Leu
           275                 280                 285
   Leu Glu Pro Thr Val Leu Pro Glu Arg Arg Leu Trp Pro Arg Tyr Leu
       290                 295                 300
   Leu Leu Gly Trp Ala Phe Pro Val Leu Phe Val Val Pro Trp Gly Phe
   305                 310                 315                 320
   Ala Arg Ala His Leu Glu Asn Thr Gly Cys Trp Thr Thr Asn Gly Asn
                   325                 330                 335
   Lys Lys Ile Trp Trp Ile Ile Arg Gly Pro Met Met Leu Cys Val Thr
               340                 345                 350
   Val Asn Phe Phe Ile Phe Leu Lys Ile Leu Lys Leu Leu Ile Ser Lys
           355                 360                 365
   Leu Lys Ala His Gln Met Cys Phe Arg Asp Tyr Lys Tyr Arg Leu Ala
       370                 375                 380
   Lys Ser Thr Leu Val Leu Ile Pro Leu Leu Gly Val His Glu Ile Leu
   385                 390                 395                 400
   Phe Ser Phe Ile Thr Asp Asp Gln Val Glu Gly Phe Ala Lys Leu Ile
                   405                 410                 415
   Arg Leu Phe Ile Gln Leu Thr Leu Ser Ser Phe His Gly Phe Leu Val
               420                 425                 430
   Ala Leu Gln Tyr Gly Phe Ala Asn Gly Glu Val Lys Ala Glu Leu Arg
           435                 440                 445
   Lys Tyr Trp Val Arg Phe Leu Leu Ala Arg His Ser Gly Cys Arg Ala
       450                 455                 460
   Cys Val Leu Gly Lys Asp Phe Arg Phe Leu Gly Lys Cys Pro Lys Lys
   465                 470                 475                 480
   Leu Ser Glu Gly Asp Gly Ala Glu Lys Leu Arg Lys Leu Gln Pro Ser
                   485                 490                 495
   Leu Asn Ser Gly Arg Leu Leu His Leu Ala Met Arg Gly Leu Gly Glu
               500                 505                 510
   Leu Gly Ala Gln Pro Gln Gln Asp His Ala Arg Trp Pro Arg Gly Ser
           515                 520                 525
   Ser Leu Ser Glu Cys Ser Glu Gly Asp Val Thr Met Ala Asn Thr Met
       530                 535                 540
   Glu Glu Ile Leu Glu Glu Ser Glu Ile
   545                 550
<210> 36
   <211> 553
   <212> PRT
   <213> Artificial Sequence
<220>
   <223> GLP 2R Variant
<400> 36
   Met Lys Leu Gly Ser Ser Arg Ala Gly Pro Gly Arg Gly Ser Ala Gly
   1               5                   10                  15
   Leu Leu Pro Gly Val His Glu Leu Pro Met Gly Ile Pro Ala Pro Trp
               20                  25                  30
   Gly Thr Ser Pro Leu Ser Phe His Arg Lys Cys Ser Leu Trp Ala Pro
           35                  40                  45
   Gly Arg Pro Phe Leu Thr Leu Val Leu Leu Val Ser Ile Lys Gln Val
       50                  55                  60
   Thr Gly Ser Leu Leu Glu Glu Thr Thr Arg Lys Trp Ala Gln Tyr Lys
   65                  70                  75                  80
   Gln Ala Cys Leu Arg Asp Leu Leu Lys Glu Pro Ser Gly Ile Phe Cys
                   85                  90                  95
   Asn Gly Thr Phe Asp Gln Tyr Val Cys Trp Pro His Ser Ser Pro Gly
               100                 105                 110
   Asn Val Ser Val Pro Cys Pro Ser Tyr Leu Pro Trp Trp Ser Glu Glu
           115                 120                 125
   Ser Ser Gly Arg Ala Tyr Arg His Cys Leu Ala Gln Gly Thr Trp Gln
       130                 135                 140
   Thr Ile Glu Asn Ala Thr Asp Ile Trp Gln Asp Asp Ser Glu Cys Ser
   145                 150                 155                 160
   Glu Asn His Ser Phe Lys Gln Asn Val Asp Arg Tyr Ala Leu Leu Ser
                   165                 170                 175
   Thr Leu Gln Leu Met Tyr Thr Val Gly Tyr Ser Phe Ser Leu Ile Ser
               180                 185                 190
   Leu Phe Leu Ala Leu Thr Leu Leu Leu Phe Leu Arg Lys Leu His Cys
           195                 200                 205
   Thr Arg Asn Tyr Ile His Met Asn Leu Phe Ala Ser Phe Ile Leu Arg
       210                 215                 220
   Thr Leu Ala Val Leu Val Lys Asp Val Val Phe Tyr Asn Ser Tyr Ser
   225                 230                 235                 240
   Lys Arg Pro Asp Asn Glu Asn Gly Trp Met Ser Tyr Leu Ser Glu Met
   245 250 255
   Ser Thr Ser Cys Arg Ser Val Gln Val Leu Leu His Tyr Phe Val Gly
   260 265 270
   Ala Asn Tyr Leu Trp Leu Leu Val Glu Gly Leu Tyr Leu His Thr Leu
   275 280 285
   Leu Glu Pro Thr Val Leu Pro Glu Arg Arg Leu Trp Pro Arg Tyr Leu
   290 295 300
   Leu Leu Gly Trp Ala Phe Pro Val Leu Phe Val Val Pro Trp Gly Phe
   305 310 315 320
   Ala Arg Ala His Leu Glu Asn Thr Gly Cys Trp Thr Thr Asn Gly Asn
   325 330 335
   Lys Lys Ile Trp Trp Ile Ile Arg Gly Pro Met Met Leu Cys Val Thr
   340 345 350
   Val Asn Phe Phe Ile Phe Leu Lys Ile Leu Lys Leu Leu Ile Ser Lys
   355 360 365
   Leu Lys Ala His Gln Met Cys Phe Arg Asp Tyr Lys Tyr Arg Leu Ala
   370 375 380
   Lys Ser Thr Leu Val Leu Ile Pro Leu Leu Gly Val His Glu Ile Leu
   385 390 395 400
   Phe Ser Phe Ile Thr Asp Asp Gln Val Glu Gly Phe Ala Lys Leu Ile
   405 410 415
   Arg Leu Phe Ile Gln Leu Thr Leu Ser Ser Phe His Gly Phe Leu Val
   420 425 430
   Ala Leu Gln Tyr Gly Phe Ala Asn Gly Glu Val Lys Ala Glu Leu Arg
   435 440 445
   Lys Tyr Trp Val Arg Phe Leu Leu Ala Arg His Ser Gly Cys Arg Ala
   450 455 460
   Cys Val Leu Gly Lys Asp Phe Arg Phe Leu Gly Lys Cys Pro Lys Lys
   465 470 475 480
   Leu Ser Glu Gly Asp Gly Ala Glu Lys Leu Arg Lys Leu Gln Pro Ser
   485 490 495
   Leu Asn Ser Gly Arg Leu Leu His Leu Ala Met Arg Gly Leu Ala Asp
   500 505 510
   Val Gly Ala Gln Pro Gln Gln Asp His Ala Arg Trp Pro Arg Gly Ser
   515 520 525
   Ser Leu Ser Glu Cys Ser Glu Gly Asp Val Thr Met Ala Asn Thr Met
   530 535 540
   Glu Glu Ile Leu Glu Glu Ser Glu Ile
   545 550
<210> 37
   <211> 59
   <212> DNA
   <213> Artificial Sequence
<220>
   <223> Primer
<400> 37
   ccaaagtata caggcgcata gcgatggttc tttctctgat gagatgaaca ccattcttg 59
<210> 38
   <211> 65
   <212> DNA
   <213> Artificial Sequence
<220>
   <223> Primer
<400> 38
   ttggtctgaa tcaaccagtt tataaagtct cgagcggcaa gattatcaag aatggtgttc 60
   atctc                                                             65
<210> 39
   <211> 27
   <212> DNA
   <213> Artificial Sequence
<220>
   <223> Primer
<400> 39
   tttgcggccg cccaaagtat acaggcg 27
<210> 40
   <211> 37
   <212> DNA
   <213> Artificial Sequence
<220>
   <223> Primer
<400> 40
   aaaggatccg tcagtgattt tggtctgaat caaccag 37
<210> 41
   <211> 59
   <212> DNA
   <213> Artificial Sequence
<220>
   <223> Primer
<400> 41
   ccaaagtata caggcgcatg gcgatggttc tttctctgat gagatgaaca ccattcttg 59
<210> 42
   <211> 59
   <212> PRT
   <213> Artificial Sequence
<220>
   <223> Mimetibody
<400> 42
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Ala Leu Val Pro Val Ser Ser Glu
           35                  40                  45
   Ser Lys Tyr Gly Pro Pro Cys Pro Pro Cys Pro
       50                  55
<210> 43
   <211> 59
   <212> PRT
   <213> Artificial Sequence
<220>
   <223> Mimetibody
<400> 43
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Ala Leu Val Ala Val Pro Ser Glu
           35                  40                  45
   Ser Lys Tyr Gly Pro Pro Cys Pro Pro Cys Pro
       50                  55
<210> 44
   <211> 59
   <212> PRT
   <213> Artificial Sequence
<220>
   <223> Mimetibody
<400> 44
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Ala Leu Val Ala Val Ser Pro Glu
           35                  40                  45
   Ser Lys Tyr Gly Pro Pro Cys Pro Pro Cys Pro
       50                  55
<210> 45
   <211> 59
   <212> PRT
   <213> Artificial Sequence
<220>
   <223> Mimetibody
<400> 45
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Ala Leu Val Ala Val Ser Ser Glu
           35                  40                  45
   Ser Lys Pro Gly Pro Pro Cys Pro Pro Cys Pro
       50                  55
<210> 46
   <211> 177
   <212> DNA
   <213> Artificial Sequence
<220>
   <223> Mimetibody
<400> 46
   catggcgatg gttctttctc tgatgagatg aacaccattc ttgataatct tgccgctcga 60
   gactttataa actggttgat tcagaccaaa atcactgacg gatccggtgg aggctccggt 120
   gccttagtcc ccgtctcctc agagtccaaa tatggtcccc catgcccacc atgcccg    177
<210> 47
   <211> 176
   <212> DNA
   <213> Artificial Sequence
<220>
   <223> Mimetibody
<400> 47
   atggcgatgg ttctttctct gatgagatga acaccattct tgataatctt gccgctcgag 60
   actttataaa ctggttgatt cagaccaaaa tcactgacgg atccggtgga ggctccggtg 120
   ccttagtcgc cgtcccctca gagtccaaat atggtccccc atgcccacca tgcccg     176
<210> 48
   <211> 177
   <212> DNA
   <213> Artificial Sequence
<220>
   <223> Mimetibody
<400> 48
   catggcgatg gttctttctc tgatgagatg aacaccattc ttgataatct tgccgctcga 60
   gactttataa actggttgat tcagaccaaa atcactgacg gatccggtgg aggctccggt 120
   gccttagtcg ccgtctcccc agagtccaaa tatggtcccc catgcccacc atgcccg    177
<210> 49
   <211> 177
   <212> DNA
   <213> Artificial Sequence
<220>
   <223> Mimetibody
<400> 49
   catggcgatg gttctttctc tgatgagatg aacaccattc ttgataatct tgccgctcga 60
   gactttataa actggttgat tcagaccaaa atcactgacg gatccggtgg aggctccggt 120
   gccttagtcg ccgtctcctc agagtccaaa cctggtcccc catgcccacc atgcccg    177
<210> 50
   <211> 33
   <212> PRT
   <213> Artificial Sequence
<220>
   <223> GLP-2 variant
<400> 50
   His Gly Asp Gly Ser Phe Ser Ser Asp Met Ser Thr Ile Leu Asp Asn
   1 5 10 15
   Leu Ala Ala Arg Asp Phe Ile Asn Trp Leu Ile Gln Thr Lys Ile Thr
   20 25 30
   Asp
<210> 51
   <211> 33
   <212> PRT
   <213> Artificial Sequence
<220>
   <223> GLP-2 variant
<400> 51
   His Gly Asp Gly Ser Phe Ser Ser Asp Val Ser Thr Ile Leu Asp Asn
   1 5 10 15
   Leu Ala Ala Arg Asp Phe Ile Asn Trp Leu Ile Gln Thr Lys Ile Thr
   20 25 30
   Asp
<210> 52
   <211> 33
   <212> PRT
   <213> Artificial Sequence
<220>
   <223> GLP-2 variant
<400> 52
   His Gly Asp Gly Ser Phe Ser Asp Glu Met Asn Thr Tyr Leu Asp Asn
   1 5 10 15
   Leu Ala Ala Arg Asp Phe Ile Asn Trp Leu Ile Gln Thr Lys Ile Thr
   20 25 30
   Asp
<210> 53
   <211> 33
   <212> PRT
   <213> Artificial Sequence
<220>
   <223> GLP-2 variant
<400> 53
   His Gly Asp Gly Ser Phe Ser Asp Glu Met Asn Thr Ile Leu Asp Gly
   1 5 10 15
   Leu Ala Ala Arg Asp Phe Ile Asn Trp Leu Ile Gln Thr Lys Ile Thr
   20 25 30
   Asp
<210> 54
   <211> 33
   <212> PRT
   <213> Artificial Sequence
<220>
   <223> GLP-2 variant
<400> 54
   His Gly Asp Gly Ser Phe Ser Asp Glu Met Asn Thr Ile Leu Asp Asn
    1               5                  10                  15
   Gln Ala Ala Arg Asp Phe Ile Asn Trp Leu Ile Gln Thr Lys Ile Thr
               20                  25                  30
   Asp
<210> 55
   <211> 33
   <212> PRT
   <213> Artificial Sequence
<220>
   <223> GLP-2 variant
<400> 55
   His Gly Asp Gly Ser Phe Ser Asp Glu Met Asn Thr Ile Leu Asp Gly
    1               5                  10                  15
   Gln Ala Ala Arg Asp Phe Ile Asn Trp Leu Ile Gln Thr Lys Ile Thr
               20                  25                  30
   Asp
<210> 56
   <211> 33
   <212> PRT
   <213> Artificial Sequence
<220>
   <223> GLP-2 variant
<400> 56
   His Gly Asp Gly Ser Phe Ser Asp Glu Met Asn Thr Ile Leu Asp Asn
    1               5                  10                  15
   Leu Ala Ala Arg Asp Phe Ile Ala Trp Leu Ile Gln Thr Lys Ile Thr
               20                  25                  30
   Asp
<210> 57
   <211> 33
   <212> PRT
   <213> Artificial Sequence
<220>
   <223> GLP-2 variant
<400> 57
   His Gly Asp Gly Ser Phe Ser Asp Glu Met Asn Thr Ile Leu Asp Asn
   1 5 10 15
   Leu Ala Ala Arg Asp Phe Ile Asn Trp Leu Val Lys Gly Lys Ile Thr
   20 25 30
   Asp
<210> 58
   <211> 59
   <212> PRT
   <213> Artificial Sequence
<220>
   <223> Mimetibody
<400> 58
   His Gly Asp Gly Ser Phe Ser Ser Asp Met Ser Thr Ile Leu Asp Asn
   1 5 10 15
   Leu Ala Ala Arg Asp Phe Ile Asn Trp Leu Ile Gln Thr Lys Ile Thr
   20 25 30
   Asp Gly Ser Gly Gly Gly Ser Gly Thr Leu Val Thr Val Ser Ser Glu
   35 40 45
   Ser Lys Tyr Gly Pro Pro Cys Pro Pro Cys Pro
   50 55
<210> 59
   <211> 59
   <212> PRT
   <213> Artificial Sequence
<220>
   <223> Mimetibody
<400> 59
   His Gly Asp Gly Ser Phe Ser Ser Asp Val Ser Thr Ile Leu Asp Asn
   1 5 10 15
   Leu Ala Ala Arg Asp Phe Ile Asn Trp Leu Ile Gln Thr Lys Ile Thr
   20 25 30
   Asp Gly Ser Gly Gly Gly Ser Gly Thr Leu Val Thr Val Ser Ser Glu
   35 40 45
   Ser Lys Tyr Gly Pro Pro Cys Pro Pro Cys Pro
   50 55
<210> 60
   <211> 59
   <212> PRT
   <213> Artificial Sequence
<220>
   <223> Mimetibody
<400> 60
   His Gly Asp Gly Ser Phe Ser Asp Glu Met Asn Thr Tyr Leu Asp Asn
   1               5                   10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro
       50                  55
<210> 61
   <211> 59
   <212> PRT
   <213> Artificial Sequence
<220>
   <223> Mimetibody
<400> 61
   His Gly Asp Gly Ser Phe Ser Asp Glu Met Asn Thr Ile Leu Asp Gly
   1               5                   10                  15
   Leu Ala Ala Arg Asp Phe Ile Asn Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro
       50                  55
<210> 62
   <211> 59
   <212> PRT
   <213> Artificial Sequence
<220>
   <223> Mimetibody
<400> 62
   His Gly Asp Gly Ser Phe Ser Asp Glu Met Asn Thr Ile Leu Asp Asn
   1               5                   10                  15
   Gln Ala Ala Arg Asp Phe Ile Asn Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro
       50                  55
<210> 63
   <211> 59
   <212> PRT
   <213> Artificial Sequence
<220>
   <223> Mimetibody
<400> 63
   His Gly Asp Gly Ser Phe Ser Asp Glu Met Asn Thr Ile Leu Asp Gly
    1               5                  10                  15
   Gln Ala Ala Arg Asp Phe Ile Asn Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro
       50                  55
<210> 64
   <211> 59
   <212> PRT
   <213> Artificial Sequence
<220>
   <223> Mimetibody
<400> 64
   His Gly Asp Gly Ser Phe Ser Asp Glu Met Asn Thr Ile Leu Asp Asn
    1               5                  10                  15
   Leu Ala Ala Arg Asp Phe Ile Ala Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro
       50                  55
<210> 65
   <211> 59
   <212> PRT
   <213> Artificial Sequence
<220>
   <223> Mimetibody
<400> 65
   His Gly Asp Gly Ser Phe Ser Asp Glu Met Asn Thr Ile Leu Asp Asn
    1               5                  10                  15
   Leu Ala Ala Arg Asp Phe Ile Asn Trp Leu Val Lys Gly Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro
       50                  55
<210> 66
   <211> 177
   <212> DNA
   <213> Artificial Sequence
<220>
   <223> Mimetibody
<400> 66
   catggcgatg gttctttctc tagtgacatg tcgaccattc ttgataatct tgccgctcga 60
   gactttataa actggttgat tcagaccaaa atcactgacg gatccggtgg aggctccggt 120
   accttagtca ccgtctcctc agagtccaaa tatggtcccc catgcccacc atgcccg    177
<210> 67
   <211> 177
   <212> DNA
   <213> Artificial Sequence
<220>
   <223> Mimetibody
<400> 67
   catggcgatg gttctttctc tagtgacgtc tcgaccattc ttgataatct tgccgctcga 60
   gactttataa actggttgat tcagaccaaa atcactgacg gatccggtgg aggctccggt 120
   accttagtca ccgtctcctc agagtccaaa tatggtcccc catgcccacc atgcccg    177
<210> 68
   <211> 177
   <212> DNA
   <213> Artificial Sequence
<220>
   <223> Mimetibody
<400> 68
   catggcgatg gttctttctc tgatgagatg aacacctatc ttgataatct tgccgctcga 60
   gactttataa actggttgat tcagaccaaa atcactgacg gatccggtgg aggctccggt 120
   accttagtca ccgtctcctc agagtccaaa tatggtcccc catgcccacc atgcccg    177
<210> 69
   <211> 177
   <212> DNA
   <213> Artificial Sequence
<220>
   <223> Mimetibody
<400> 69
   catggcgatg gttctttctc tgatgagatg aacaccattc ttgatggtct tgccgctcga 60
   gactttataa actggttgat tcagaccaaa atcactgacg gatccggtgg aggctccggt 120
   accttagtca ccgtctcctc agagtccaaa tatggtcccc catgcccacc atgcccg    177
<210> 70
   <211> 177
   <212> DNA
   <213> Artificial Sequence
<220>
   <223> Mimetibody
<400> 70
   catggcgatg gttctttctc tgatgagatg aacaccattc ttgataatca ggccgctcga 60
   gactttataa actggttgat tcagaccaaa atcactgacg gatccggtgg aggctccggt 120
   accttagtca ccgtctcctc agagtccaaa tatggtcccc catgcccacc atgcccg    177
<210> 71
   <211> 177
   <212> DNA
   <213> Artificial Sequence
<220>
   <223> Mimetibody
<400> 71
   catggcgatg gttctttctc tgatgagatg aacaccattc ttgatggcca ggccgctcga 60
   gactttataa actggttgat tcagaccaaa atcactgacg gatccggtgg aggctccggt 120
   accttagtca ccgtctcctc agagtccaaa tatggtcccc catgcccacc atgcccg    177
<210> 72
   <211> 177
   <212> DNA
   <213> Artificial Sequence
<220>
   <223> Mimetibody
<400> 72
   catggcgatg gttctttctc tgatgagatg aacaccattc ttgataatct tgccgctcga 60
   gactttatag cctggttgat tcagaccaaa atcactgacg gatccggtgg aggctccggt 120
   accttagtca ccgtctcctc agagtccaaa tatggtcccc catgcccacc atgcccg    177
<210> 73
   <211> 177
   <212> DNA
   <213> Artificial Sequence
<220>
   <223> Mimetibody
<400> 73
   catggcgatg gttctttctc tgatgagatg aacaccattc ttgataatct tgccgctcga 60
   gactttataa actggttggt taagggcaaa atcactgacg gatccggtgg aggctccggt 120
   accttagtca ccgtctcctc agagtccaaa tatggtcccc catgcccacc atgcccg    177
<210> 74
   <211> 33
   <212> PRT
   <213> Artificial Sequence
<220>
   <223> GLP-2 Peptide with A2G, M10V mutation
<400> 74
   His Gly Asp Gly Ser Phe Ser Asp Glu Val Asn Thr Ile Leu Asp Asn
   1               5                   10                  15
   Leu Ala Ala Arg Asp Phe Ile Asn Trp Leu Ile Gln Thr Lys Ile
               20                  25                  30
   Thr Asp
<210> 75
   <211> 276
   <212> PRT
   <213> Artificial Sequence
<220>
   <223> GLP-2 Mimetibody amino acid sequence with A2G, L17Q mutation
<400> 75
   His Gly Asp Gly Ser Phe Ser Asp Glu Met Asn Thr Ile Leu Asp Asn
   1               5                   10                  15
   Gln Ala Ala Arg Asp Phe Ile Asn Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro Ala Pro Glu Ala Ala
       50                  55                  60
   Gly Gly Pro Ser Val Phe Leu Phe Pro Pro Lys Pro Lys Asp Thr Leu
   65                  70                  75                  80
   Met Ile Ser Arg Thr Pro Glu Val Thr Cys Val Val Val Asp Val Ser
                   85                  90                  95
   Gln Glu Asp Pro Glu Val Gln Phe Asn Trp Tyr Val Asp Gly Val Glu
               100                 105                 110
   Val His Asn Ala Lys Thr Lys Pro Arg Glu Glu Gln Phe Asn Ser Thr
           115                 120                 125
   Tyr Arg Val Val Ser Val Leu Thr Val Leu His Gln Asp Trp Leu Asn
       130                 135                 140
   Gly Lys Glu Tyr Lys Cys Lys Val Ser Asn Lys Gly Leu Pro Ser Ser
   145                 150                 155                 160
   Ile Glu Lys Thr Ile Ser Lys Ala Lys Gly Gln Pro Arg Glu Pro Gln
                   165                 170                 175
   Val Tyr Thr Leu Pro Pro Ser Gln Glu Glu Met Thr Lys Asn Gln Val
               180                 185                 190
   Ser Leu Thr Cys Leu Val Lys Gly Phe Tyr Pro Ser Asp Ile Ala Val
           195                 200                 205
   Glu Trp Glu Ser Asn Gly Gln Pro Glu Asn Asn Tyr Lys Thr Thr Pro
       210                 215                 220
   Pro Val Leu Asp Ser Asp Gly Ser Phe Phe Leu Tyr Ser Arg Leu Thr
   225                 230                 235                 240
   Val Asp Lys Ser Arg Trp Gln Glu Gly Asn Val Phe Ser Cys Ser Val
                   245                 250                 255
   Met His Glu Ala Leu His Asn His Tyr Thr Gln Lys Ser Leu Ser Leu
               260                 265                 270
   Ser Leu Gly Lys
           275
<210> 76
   <211> 828
   <212> DNA
   <213> Artificial Sequence
<220>
   <223> GLP-2 Mimetibody nucleic acid sequence with A2G, L17Q mutation
<400> 76
   catggcgatg gttctttctc tgatgagatg aacaccattc ttgataatca ggccgctcga 60
   gactttataa actggttgat tcagaccaaa atcactgacg gatccggtgg aggctccggt 120
   accttagtca ccgtctcctc agagtccaaa tatggtcccc catgcccacc atgcccggcg 180
   cctgaggccg ccgggggacc atcagtcttc ctgttccccc caaaacccaa ggacactctc 240
   atgatctccc ggacccctga ggtcacgtgc gtggtggtgg acgtgagcca ggaagacccc 300
   gaggtccagt tcaactggta cgtggatggc gtggaggtgc ataatgccaa gacaaagccg 360
   cgggaggagc agttcaacag cacgtaccgt gtggtcagcg tcctcaccgt cctgcaccag 420
   gactggctga acggcaagga gtacaagtgc aaggtctcca acaaaggcct cccgtcctcc 480
   atcgagaaaa ccatctccaa agccaaaggg cagcctcgag agccacaggt gtacaccctg 540
   cccccatccc aggaggagat gaccaagaac caggtcagcc tgacctgcct ggtcaaaggc 600
   ttctacccca gcgacatcgc cgtggagtgg gagagcaatg ggcagccgga gaacaactac 660
   aagaccacgc ctcccgtgct ggactccgac ggctccttct tcctctacag caggctaacc 720
   gtggacaaga gcaggtggca ggaggggaat gtcttctcat gctccgtgat gcatgaggct 780
   ctgcacaacc actacacaca gaaaagcttg tccctgtctc tgggtaaa             828
<210> 77
   <211> 276
   <212> PRT
   <213> Artificial Sequence
<220>
   <223> GLP-2 Mimetibody nucleic acid sequence with A2G, N16G, L17Q mutation
<400> 77
   His Gly Asp Gly Ser Phe Ser Asp Glu Met Asn Thr Ile Leu Asp Gly
   1               5                   10                  15
   Gln Ala Ala Arg Asp Phe Ile Asn Trp Leu Ile Gln Thr Lys Ile Thr
               20                  25                  30
   Asp Gly Ser Gly Gly Gly Ser Gly Thr Leu Val Thr Val Ser Ser Glu
           35                  40                  45
   Ser Lys Tyr Gly Pro Pro Cys Pro Pro Cys Pro Ala Pro Glu Ala Ala
       50                  55                  60
   Gly Gly Pro Ser Val Phe Leu Phe Pro Pro Lys Pro Lys Asp Thr Leu
   65                  70                  75                  80
   Met Ile Ser Arg Thr Pro Glu Val Thr Cys Val Val Val Asp Val Ser
                   85                  90                  95
   Gln Glu Asp Pro Glu Val Gln Phe Asn Trp Tyr Val Asp Gly Val Glu
               100                 105                 110
   Val His Asn Ala Lys Thr Lys Pro Arg Glu Glu Gln Phe Asn Ser Thr
           115                 120                 125
   Tyr Arg Val Val Ser Val Leu Thr Val Leu His Gln Asp Trp Leu Asn
       130                 135                 140
   Gly Lys Glu Tyr Lys Cys Lys Val Ser Asn Lys Gly Leu Pro Ser Ser
   145                 150                 155                 160
   Ile Glu Lys Thr Ile Ser Lys Ala Lys Gly Gln Pro Arg Glu Pro Gln
                   165                 170                 175
   Val Tyr Thr Leu Pro Pro Ser Gln Glu Glu Met Thr Lys Asn Gln Val
               180                 185                 190
   Ser Leu Thr Cys Leu Val Lys Gly Phe Tyr Pro Ser Asp Ile Ala Val
           195                 200                 205
   Glu Trp Glu Ser Asn Gly Gln Pro Glu Asn Asn Tyr Lys Thr Thr Pro
       210                 215                 220
   Pro Val Leu Asp Ser Asp Gly Ser Phe Phe Leu Tyr Ser Arg Leu Thr
   225                 230                 235                 240
   Val Asp Lys Ser Arg Trp Gln Glu Gly Asn Val Phe Ser Cys Ser Val
                   245                 250                 255
   Met His Glu Ala Leu His Asn His Tyr Thr Gln Lys Ser Leu Ser Leu
               260                 265                 270
   Ser Leu Gly Lys
           275
<210> 78
   <211> 828
   <212> DNA
   <213> Artificial Sequence
<220>
   <223> GLP-2 Mimetibody nucleic acid sequence with A2G, N16G, L17Q mutation
<400> 78
   catggcgatg gttctttctc tgatgagatg aacaccattc ttgatggcca ggccgctcga 60
   gactttataa actggttgat tcagaccaaa atcactgacg gatccggtgg aggctccggt 120
   accttagtca ccgtctcctc agagtccaaa tatggtcccc catgcccacc atgcccggcg 180
   cctgaggccg ccgggggacc atcagtcttc ctgttccccc caaaacccaa ggacactctc 240
   atgatctccc ggacccctga ggtcacgtgc gtggtggtgg acgtgagcca ggaagacccc 300
   gaggtccagt tcaactggta cgtggatggc gtggaggtgc ataatgccaa gacaaagccg 360
   cgggaggagc agttcaacag cacgtaccgt gtggtcagcg tcctcaccgt cctgcaccag 420
   gactggctga acggcaagga gtacaagtgc aaggtctcca acaaaggcct cccgtcctcc 480
   atcgagaaaa ccatctccaa agccaaaggg cagcctcgag agccacaggt gtacaccctg 540
   cccccatccc aggaggagat gaccaagaac caggtcagcc tgacctgcct ggtcaaaggc 600
   ttctacccca gcgacatcgc cgtggagtgg gagagcaatg ggcagccgga gaacaactac 660
   aagaccacgc ctcccgtgct ggactccgac ggctccttct tcctctacag caggctaacc 720
   gtggacaaga gcaggtggca ggaggggaat gtcttctcat gctccgtgat gcatgaggct 780
   ctgcacaacc actacacaca gaaaagcttg tccctgtctc tgggtaaa 828
<210> 79
   <211> 30
   <212> PRT
   <213> Artificial Sequence
<220>
   <223> GLP-1 Mimetibody amino acid sequence
<400> 79
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 80
   <211> 280
   <212> PRT
   <213> Artificial Sequence
<220>
   <223> Human A2G-GLP2 peptide in murine IgG2a scaffold
<400> 1